# EUROPÄISCHE PATENTSCHRIFT

(11) **EP 2 826 033 B1**
(45) Veröffentlichungstag und Bekanntmachung des Hinweises auf die Patenterteilung: **10.01.2024**
(21) Anmeldenummer: 13711322.1
(22) Anmeldetag: 12.03.2013
(51) Int. Cl.: G09F 3/03

(54) **SICHERHEITSPLOMBE**
SECURITY SEAL
PLOMB DE SÉCURITÉ

(30) Priorität: 14.03.2012 DE 102012004961
(43) Veröffentlichungstag der Anmeldung: 21.01.2015
(73) Patentinhaber: Aesculap AG, 78532 Tuttlingen (DE)
(72) Erfinder: WEIßHAUPT, Dieter, 78194 Immendingen (DE); THOMAS, Stefan, 78532 Tuttlingen (DE); SCHUSTER, Stefan, 78048 Villingen-Schwenningen (DE); ELISCH, Andreas, 78713 Schramberg (DE)
(74) Vertreter: Koller, Tobias Kilian
(86) Internationale Anmeldenummer: PCT/EP2013/054944
(87) Internationale Veröffentlichungsnummer: WO 2013/135658

(56) Entgegenhaltungen:
- DE-U1- 9 405 787
- US-A- 3 990 131
- US-A- 5 651 652
- US-A1- 2004 104 230
- US-A1- 2011 253 579

## Beschreibung

Die vorliegende Erfindung betrifft eine neuartige Sicherheitsplombe, einen Sicherheitsverschluss mit einer solchen Sicherheitsplombe sowie einen Sicherheitsbehälter und einen Sterilisiercontainer mit einem solchen Sicherheitsverschluss.

Plomben werden eingesetzt, um kontrollieren zu können, ob ein Verschluss geöffnet oder eine Verbindung getrennt wurde. So werden Plomben zum Beispiel beim Zoll eingesetzt, um sicher zu stellen, dass beispielsweise ein Hochseecontainer genau die Waren enthält, die auf den zugehörigen Zollpapieren aufgeführt sind. Dazu verwendet der Zoll Drahtschlaufen, deren Enden mit einer Siegelplombe verbunden werden. Das Siegel bietet zudem die Möglichkeit, zu überprüfen, ob eine Versiegelung durch eine neue ersetzt wurde. Im Bereich der Medizin werden Plomben zum Beispiel bei Sterilisiercontainern eingesetzt, um kontrollieren zu können, ob ein solcher Sterilisiercontainer nach der Sterilisation bereits einmal geöffnet wurde und die darin enthaltenen Instrumente noch mit Sicherheit steril sind. Dazu werden verschiedene Arten von Plomben angeboten.

Eine sehr einfache Art von Plomben für Sterilisiercontainer ist in dem Dokument DE 9405787 U1 gezeigt. Diese Plombe besteht aus einem Karton, der derart geschlitzt ist, dass ein mittlerer Finger entsteht. Dieser Finger wird durch eine Art Öse an der Verschlusslasche des Containerdeckels geschoben und an seinem freien Ende mit den beiden Randbereichen der Plombe und einem Teil der Containerwanne verklebt. Wird nun die Verschlusslasche geöffnet, reist der mittlere Finger der Plombe ab und zeigt so an, dass der Verschluss bereits einmal geöffnet wurde.

Eine andere einfache Art von Plomben arbeitet nach dem Prinzip von Kabelbindern, also einer Schlaufe aus Kunststoff, die durch je eine Öse an dem Containerdeckel und der Containerwanne geführt und dann geschlossen wird. Geschlossen wird diese Art von Plombe indem ihr eines Ende, das mit einer oder mehreren Vertiefungen ausgestattet ist, deren zum proximalen Ende der Schlaufe zeigende Flanken angeschrägt sind, während die zum distalen Ende zeigenden Flanken steil angestellt sind. Am anderen Ende der Schlaufe ist ein Kopf mit einem Durchgangsloch vorgesehen, in das eine federnde Rastnase vorsteht. Das proximale Ende der Schlaufe wird so in das Durchgangsloch eingeführt, dass die Rastnase in die Vertiefung einrastet. Durch das steile Anstellen der einen Flanke der Vertiefung und entsprechende Ausbildung der Rastnase kann sichergestellt werden, dass das proximale Ende der Schlaufe leicht in das Durchgangsloch eingeschoben werden kann, aber ein Ziehen an der Schlaufe nicht dazu führt, dass das proximale Ende aus dem Durchgangsloch entweicht. Zusätzlich können an solch einer Schlaufe Beschriftungsfelder oder ähnliches vorgesehen sein. Diese Art von Plombe soll nur geöffnet werden können, indem sie zerstört wird, d.h. indem die Schlaufe an einer Stelle durchtrennt wird.

Das Problem dieser Art von Plomben ist, dass sie nicht gegen mutwillige Manipulation geschützt sind. Mit einem dünnen Gegenstand kann man nämlich in den Kopf der Plombe einfahren und die federnde Rastnase entgegen der Federwirkung aus der Vertiefung in dem proximalen Ende der Plombe heraus verformen und somit die Plombe öffnen, ohne sie zu zerstören. Man kann sie danach sogar wieder verwenden, sodass sie ihre Funktion der Sicherstellung eines Originalzustands nicht mehr erfüllen kann.

Zudem hat man im klinischen Bereich noch das Problem, dass eine ordnungsgemäß geöffnete Plombe, also eine zerstörte Plombe nur noch lose an den beiden Ösen des Containers hängt und leicht abfällt, wenn man den Deckel von der Containerwanne abhebt. Da der Boden eines Operationssaals als unsteril gilt, kann eine herunter gefallene Plombe nicht einfach aufgehoben werden. Da oft mehrere Container eingesetzt werden, können daher häufig mehrere Plomben auf den Boden fallen und den sicheren Stand und Tritt und somit die Konzentration des Personals beeinträchtigen.

Ein anderes Sicherungssystem für Sterilisiercontainer besteht aus biegbaren einer Karte, die seitlich so in einen Schlitz gesteckt wird, dass ihr freies Ende vor der Verschlusslasche des Containerdeckels liegt. Zudem verfügt das eingeschobene Ende der Karte über spezielle Aussparungen bzw. Löcher, die in dem Schlitz in zugehörige Vorsprünge einrastet, sodass die Karte zumindest bei verschlossenem Container nicht einfach aus dem Schlitz entfernt und wieder eingeführt werden kann. Wird nun die Verschlusslasche geöffnet, biegt sich die Karte und schnappt dann hinter der Verschlusslasche in ihre Ausgangslage zurück. Wenn die Verschlusslasche nun wieder geschlossen wird, liegt die Karte hinter der Verschlusslasche und nicht mehr vor ihr, sodass angezeigt wird, dass der Container bereits geöffnet wurde. Allerdings ist der Mechanismus, der einen Austausch der Karte für einen folgenden Sterilisationsvorgang ermöglicht, relativ kompliziert und damit teuer und störanfällig und wirkt sich negativ auf das Sterilisationsergebnis aus. Darüber hinaus ist auch diese Sicherheitseinrichtung nicht manipulationssicher. Mit einem langen, dünnen Gegenstand kann die Karte vor dem erneuten Verschließen der Verschlusslasche nach vorn weggebogen werden, sodass sie anschließend doch wieder vor der Verschlusslasche liegt.

In dem Dokument US 2004/01 04230 A1 ist ein Verriegelungszapfen offenbart, der über einen Fuß und einen Kopf verfügt. Der Fuß weist vier Vorsprünge auf, die zu ihrem distalen Ende hin eine Anlaufschräge aufweisen und zu ihrem proximalen Ende hin eine Stufe. Der Kopf weist einen Griffabschnitt auf, mit dessen Hilfe der Verriegelungszapfen in eine Sicherungsöffnung eingeführt, d.h. eingepresst, werden kann Der Kopf überdeckt dabei einen Teil des zu verriegelnden Gegenstands. Wird der zu verriegelnde Gegenstand nun von der Sicherungsöffnung entfernt, reist der Kopf des Verriegelungszapfens von dessen Fuß ab und zeigt so an, dass die Verriegelung bzw. Sicherung gelöst wurde.

In der Veröffentlichung US 2011/0253579 A1 ist ein Montageelement gezeigt, mit dessen Hilfe Produkte an einem Display befestigt werden können. Dieses Montageelement weist einen Fuß und einen Kopf auf. Der Fuß hat einen runden Querschnitt zwei gegenüberliegende Vorsprünge und bildet eine Art Schlüssel, der durch ein Loch in dem Display in ein entsprechendes Schlüsselloch in dem Produkt eingeführt werden kann. Anschließend wird das Montageelement gedreht und verankert so das Produkt an dem Display. Um das Montageelement leichter drehen zu können, verfügt der Kopf über zwei Laschen, die scharnierartig umgeklappt werden können, um eine vergrößerte Angriffsfläche zu bilden. Außerdem kann ein Sicherungsstreifen durch die Laschen mehrerer Montageelemente hindurchgeführt werden und so ein unbeabsichtigtes Drehen und somit Lösen der Montageelemente zu verhindern.

Ein weiteres großes Problem der bislang bekannten Plomben und Sicherheitsmechanismen ist, dass man oft nicht auf den ersten Blick erkennen kann, ob diese bereits entwertet bzw. zerstört sind, oder nicht. Beispielsweise bei einer Plombe in Schlaufenform kann eine zerstörte Plombe in die Ösen an dem Container wieder eingefädelt werden und ein Anwender mit geringer Aufmerksamkeit wird nicht unbedingt bemerken, dass die Öse bereits zerstört und damit entwertet ist.

Daher ist es eine Aufgabe der vorliegenden Erfindung, eine Sicherheitsplombe bereit zu stellen, bei der durch die Entwertung keine Teile der Plombe oder die gesamte Plombe von dem verplombten Verschluss abfallen kann.

Eine weitere Aufgabe der vorliegenden Erfindung ist es, eine Sicherheitsplombe bereit zu stellen, die ein Schließen des verplombten Verschlusses nach einem Entwerten der Plombe verhindert.

Eine weitere Aufgabe der vorliegenden Erfindung ist es, eine Sicherheitsplombe bereit zu stellen, die gegen unbeabsichtigte Entwertung durch andere Einwirkungen als das Öffnen des verplombten Verschlusses geschützt ist.

Eine weitere Aufgabe der vorliegenden Erfindung ist es, eine Sicherheitsplombe bereit zu stellen, die nur entfernt werden kann, wenn der verplombte Verschluss bereits geöffnet wurde. Weiterhin soll die Sicherheitsplombe dann ohne großen Kraftaufwand und ohne viel Feingefühl entfernbar sein.

Eine weitere Aufgabe der vorliegenden Erfindung ist es, einen Sicherheitsverschluss bereit zu stellen, der an die Verwendung einer erfindungsgemäßen Sicherheitsplombe angepasst ist.

Eine weitere Aufgabe der vorliegenden Erfindung ist es, einen Sicherheitsbehälter und insbesondere einen Sterilisiercontainer bereit zu stellen, der einen erfindungsgemäßen Sicherheitsverschluss aufweist, welcher an die Verwendung einer erfindungsgemäßen Sicherheitsplombe angepasst ist.

Um diese Aufgaben der Erfindung zu lösen wird eine Sicherheitsplombe nach einem der Ansprüche 1 bis 8, ein Sicherheitsverschluss nach einem der Ansprüche 9 bis 12, ein Sicherheitsbehälter nach Anspruch 13 und ein Sterilisiercontainer nach einem der Ansprüche 14 und 15 bereit gestellt.

Gemäß einem ersten Aspekt der vorliegenden Erfindung weist eine Sicherheitsplombe einen Plombenfuß mit mindestens zwei Rastvorsprüngen und einen mit dem Plombenfuß verbundenen Plombenkopf mit wenigstens einer Sperrlasche auf. Jede Sperrlasche ist dabei über wenigstens ein Scharnierelement mit dem Plombenkopf bewegbar verbunden. Der Plombenfuß ist mit zwei Schlitzen ausgebildet, wodurch das distale Ende des Plombenfußes nach innen verformt werden kann.

Auf diese Weise kann die Sicherheitsplombe in eine entsprechende Aufnahme an einem ersten Teil eines Verschlusses angebracht und fixiert werden. Der Rastvorsprung gewährleistet ein schnelles und einfaches Anbringen der Sicherheitsplombe an einer entsprechenden Aufnahme. Die Sperrlasche sperrt dabei den zweiten Teil des Verschlusses, sodass der Verschluss nicht geöffnet werden kann. In den meisten Fällen wird es sich um einen Verschluss handeln, bei dem sich ein Verschlussteil senkrecht von dem anderen Verschlussteil (d.h. die Verschlussteile bleiben im Wesentlichen parallel zueinander) oder gegenüber dem anderen Verschlussteil schwenkend (d.h. durch Drehung um eine Drehachse, die in der Verschlussebene liegt) entfernt, um den Verschluss zu öffnen. Es ist aber auch möglich, dass diese Sicherheitsplombe an einem Verschluss vorgesehen wird, bei dem sich ein Verschlussteil gegenüber dem anderen in der Verschlussebene verschiebt und/oder verdreht (die Verschlussteile bleiben parallel zueinander, eine Verdrehung findet ggf. um eine Achse statt, die senkrecht auf die Verschlussebene steht). Dazu kann an der Sperrlasche ein Vorsprung vorgesehen sein, der in das Verschlussteil eingreift, an dem die Sicherheitsplombe nicht angebracht ist. Dieser Vorsprung greift in eine entsprechende Vertiefung in dem gegriffenen Verschlussteil ein. Wenn nun eine Verschiebung stattfindet, wird der Eingriff des Vorsprungs in die Vertiefung mit Hilfe des Scharnierelements aufgehoben.

Gemäß einer vorteilhaften Weiterentwicklung des ersten Aspekts der vorliegenden Erfindung ist das wenigstens eine Scharnierelement zumindest teilweise plastisch verformbar.

Auf diese Weise kann sichergestellt werden, dass die Sperrlasche nach einem Öffnen des jeweiligen Verschlusses nicht mehr vollständig in ihre Ausgangsposition zurückkehren kann. Eine plastische Verformung mag teilweise rückgängig gemacht werden können, aber es ist nicht möglich, diese vollständig rückgängig zu machen. Es werden mindestens sichtbare Verformungen der Oberflächen in an den Biegebereichen des oder der Scharnierelemente verbleiben. Auf diese Weise kann man der Plombe ansehen, dass der zugehörige Verschluss bereits mindestens einmal geöffnet wurde. Die Plombe ist daher durch den ersten Einsatz entwertet worden. Besonders vorteilhaft ist die plastische Verformung, um bewussten Manipulationen der Plombe bzw. des verplombten Verschlusses entgegen zu wirken. Weiter vorteilhaft ist es, wenn die Verformung nicht ausschließlich plastisch erfolgt sondern auch einen elastischen Anteil aufweist. Dadurch kann ein Teil der Sicherheitslasche beim Öffnen des zugehörigen Verschlusses zunächst durch das gesperrte Verschlussteil aus der Bewegungskurve heraus gedrängt werden und danach wieder in dessen Bewegungskurve eintreten.

Bei einer relativen Bewegung der Verschlusselemente innerhalb der Verschlussebene bzw. senkrecht zur Längsachse der Sicherheitsplombe zum Öffnen des Verschlusses und bei einer Sperrlasche mit einem Vorsprung zum Eingriff in das gesperrte Verschlussteil stellt sich die Interaktion des Sperrlasche mit der Bewegungskurve des Verschlussteils folgendermaßen dar. Die Bewegungsrichtung des gesperrten Verschlussteils relativ zu der Sicherheitsplombe und damit zu dem anderen Verschlussteil ist im Wesentlichen parallel zu der Erstreckungsrichtung des Scharnierelements im Ausgangszustand. Schräge Kontaktflächen an dem Verschlussteil und an der Sperrlasche sorgen dafür, dass bei einer Entfernung der Verschlussteile voneinander der Vorsprung an der Sperrlasche leicht in einer Richtung im Wesentlichen senkrecht zu der Erstreckungsrichtung des Scharnierelements aus der Vertiefung in dem gesperrten Verschlussteil heraus tritt, wobei schräg in diesem Zusammenhang bedeutet, dass der Winkel zwischen diesen Flächen und der Bewegungsrichtung nicht allzu groß ist, zum Beispiel 45° ± 10° (keine absoluten Bereichsgrenzen). Dabei wird das Scharnierelement elastisch und plastisch verformt. Ist das vormals gesperrte Verschlussteil vollständig entfernt, verformt sich die Sperrlasche elastisch zurück, sodass nur die plastische Verformung erhalten bleibt. Versucht man nun, das vormals gesperrte Verschlussteil in die Schließstellung zurück zu führen, so stoßen zumindest die beiden Flächen des Vorsprungs an der Sperrlasche und des vormals gesperrten Bauteils an den Flächen aneinander, die zu den schrägen Kontaktfläche jeweils in Bewegungsrichtung an den entgegen gesetzten Seiten des jeweiligen Bauteils vorgesehen sind. Nehmen nun diese Flächen eine im Wesentlichen senkrechte Lage gegenüber der Bewegungsrichtung ein, so weicht die Sperrlasche nicht aus und verhindert somit, dass die vormals gesperrte Verschlusslasche in ihre Ausgangsposition bzw. gesperrte Position zurückkehren kann.

Bei einer relativen Bewegung der Verschlusselemente aus der Verschlussebene heraus zum Öffnen des Verschlusses bzw. parallel zur Längsachse der Sicherheitsplombe zum Öffnen des Verschlusses stellt sich die Interaktion der Sperrlasche mit der Bewegungskurve des Verschlussteils folgendermaßen dar. Die Sperrlasche sperrt das gesperrte Verschlussteil, indem sie dieses zumindest teilweise in der Bewegungsrichtung überlappt. Wird nun das Verschlussteil aus der Schließstellung heraus bewegt, also angehoben oder verschwenkt, drängt das gesperrte Verschlussteil die Sperrlasche unter elastischer und plastischer Verformung des mindestens einen Scharnierelements aus der Bewegungskurve des Verschlussteils. Wenn das Verschlussteil vollständig von der Sicherheitsplombe entfernt ist, kehrt die Sperrlasche so weit in die Bahnkurve des vormals gesperrten Verschlussteils zurück, dass die induzierte elastische Verformung rückgängig gemacht wird. In diesem Fall ist die Erstreckungsrichtung des mindestens einen Scharnierelements im Wesentlichen senkrecht zu der Bewegungsrichtung des Verschlussteils, zumindest in dem Bereich, in dem ein Kontakt zwischen Sicherheitsplombe und gesperrtem Verschlussteil vorliegt. Versucht nun das vormals gesperrte Verschlussteil in die Schließstellung zurück zu kehren, kommt es in Anlage an die Sperrlasche. Durch geeignete Ausbildung der entsprechenden Anlageflächen kann nun verhindert werden, dass die Sperrlasche dem vormals gesperrten Verschlussteil ausweicht und somit dessen Bewegungskurve frei gibt. Vielmehr kann die Sicherheitsplombe derart gestaltet sein, dass der Kontakt zwischen Sperrlasche und Verschlussteil dazu führt, dass die Sperrlasche noch weiter in die Bewegungskurve des Verschlussteils gedrängt wird. Zudem kann an der Sicherheitsplombe auch ein Anschlag oder eine Anlagefläche vorgesehen sein, der/die verhindert, dass die Sperrlasche in die entgegen gesetzte Richtung der ersten Verformung aus der Bahnkurve heraus gedrängt wird. Üblicherweise wird dies jedoch bereits durch den Plombenkopf oder den Plombenfuß selbst gewährleistet oder durch ein Anliegen der Sperrlasche an einer Oberfläche des ersten Verschlussteils.

Gemäß einer vorteilhaften Weiterentwicklung des ersten Aspekts der vorliegenden Erfindung ist die Sicherheitsplombe einstückig vorzugsweise aus Kunststoff ausgebildet. Eine einstückige Sicherheitsplombe hat den Vorteil, dass keine Montageschritte erforderlich sind. Als Werkstoff eignet sich besonders Kunststoff, da sich dieser leicht in die gewünschte Form bringen lässt, beispielsweise durch Spritzguss, und da sich bei einem Gussverfahren mit Zwangsentformung auch gewisse Hinterschneidungen an der Sicherheitsplombe ausbilden lassen. Alternativ kann die Sicherheitsplombe auch aus Metall oder einem Verbundwerkstoff ausgebildet sein. Wenn nun die Sicherheitsplombe einstückig ausgebildet ist, wird das mindestens eine Scharnierelement bei einer Auslenkung der Sperrlasche verbogen. Dabei wird zumindest ein Teil der Sperrlasche plastisch verformt, sodass es bei einer Sicherheitsplombe aus Kunststoff zum so genannten Weißbruch kommt. Diese plastische Verformung sorgt dafür, dass die Sperrlasche nicht mehr vollständig in ihre Ausgangsposition zurück gebracht werden kann. Bei einer Sicherheitsplombe aus Kunststoff kann zudem durch geeignete Auswahl der Materialeigenschaften verhindert werden, dass die Sperrlasche beim Biegen des mindestens einen Scharnierelements von der Sicherheitsplombe abbricht bzw. abreißt. Auf diese Weise ist selbst die entwertete Sicherheitsplombe einstückig, und nicht zerstört und in mehrere Teile zerlegt, sodass kein Teil der Sicherheitsplombe abgeht und anschließend eingesammelt werden muss.

Gemäß einer besonders vorteilhaften Weiterentwicklung des ersten Aspekts der vorliegenden Erfindung weist die Sicherheitsplombe zwei vorzugsweise in radialer Richtung gegenüber liegenden Sperrlaschen auf. Auf diese Weise hat der Plombenkopf eine Art Schmetterlingsgestalt. Eine so ausgebildete Plombe eignet sich besonders für Verschlüsse, in denen die Plombe in ein Durchgangsloch eingeführt wird, welches in einem ersten Verschlussteil vorgesehen ist. Nach dem Öffnen des Verschlusses und dem damit einhergehenden Aufbiegen der beiden Sperrlaschen kommen die Sperrlaschen bei dem Versuch, des erneuten Schließens des Verschlusses mit gegenüber liegenden Rändern des Durchgangsloches in Kontakt. Dies führt aufgrund der Geometrie der Sicherheitsplombe und der Speerlaschen zu einem Verkeilen der Sicherheitsplombe in dem Durchgangsloch, sodass auf diese Weise besonders sichergestellt werden kann, dass der Verschluss nicht mehr geschlossen werden kann, bis die Sicherheitsplombe von dem Verschluss entfernt wird.

Gemäß einer vorteilhaften Weiterentwicklung des ersten Aspekts der vorliegenden Erfindung ist jede Sperrlasche der Sicherheitsplombe mit zwei Scharnierelementen vorzugsweise seitlich am Plombenkopf vorgesehen. Eine Verbindung jeder Sperrlasche mit zwei Scharnierelementen stellt eine stabile und sichere Verbindung dar. Zudem kann so verhindert werden, dass sich die Sperrlasche gegenüber dem Plombenkopf um eine Achse parallel zu dem Scharnierelement verdreht. Wenn die Scharnierelemente seitlich am Plombenkopf vorgesehen sind, können die Scharnierelemente länger ausgebildet sein, sodass die zugehörige Sperrlasche einen Bewegungspfad mit größerem Krümmungsradius aufweist. Dies führt dazu, dass eine entwertete Plombe auch mit geringerer Aufmerksamkeit des Nutzers leichter zu erkennen ist, da die Sperrlasche eine Position einnimmt, die weiter von ihrer Ausgangsposition entfernt ist.

Gemäß einer besonders vorteilhaften Weiterentwicklung des ersten Aspekts der vorliegenden Erfindung weist der Plombenfuß einen im Wesentlichen kreisförmigen Querschnitt auf. Dies ermöglicht eine Sicherheitsplombe, die geradlinig in eine entsprechende Plombenfußaufnahme eingeschoben bzw. eingesteckt werden kann und dann, wenn der Verschluss geöffnet ist, um die Plombenlängsachse gedreht werden kann, um die Hinterschneidung des Rastvorsprunges mit der Plombenfußaufnahme aufzuheben und die Plombe aus der Plombenfußaufnahme zu entnehmen. Alternativ dazu kann der Plombenfuß auch rechteckig ausgebildet sein. Dann kann die Sicherheitsplombe beispielsweise senkrecht zur Plombenlängsachse verschoben werden, um die Hinterschneidung zwischen Rastvorsprung und Aufnahme aufzuheben.

Gemäß einer weiteren vorteilhaften Weiterentwicklung des ersten Aspekts der vorliegenden Erfindung steht jede Sperrlasche zumindest teilweise gegenüber dem Plombenfuß radial vor. Auf diese Weise kann die Mantelfläche des Plombenfußes an dem Rand der Plombenfußaufnahme anliegen und so einen sicheren Halt der Sicherheitsplombe in der Aufnahme gewährleisten.

Gemäß einer vorteilhaften Weiterentwicklung des ersten Aspekts der vorliegenden Erfindung stehen die mindestens zwei Rastvorsprünge in radialer Richtung vom Plombenfuß nach außen vor. Eine solche Sicherheitsplombe benötigt, wenn der Plombenfuß einen kreisförmigen Querschnitt aufweist, als Aufnahme lediglich ein kreisförmiges Loch mit mindestens zwei radialen Aufweitungen, durch die die mindestens zwei Rastvorsprünge passt, sodass die Sicherheitsplombe bei Bedarf leicht von der Aufnahme entfernt werden kann. Sinnvoller Weise entspricht die Anzahl der radialen Aufweitungen mindestens der Anzahl von Rastvorsprüngen, welche nach außen vorstehen. Radiale Aufweisung bedeutet in diesem Zusammenhang, dass der Durchmesser der Aufnahme im Bereich der Aufweitung größer als an einer Stelle ist, an der keine radiale Aufweitung ausgebildet ist.

Gemäß einer vorteilhaften Weiterentwicklung des ersten Aspekts der vorliegenden Erfindung ist am Plombenkopf oder am Plombenfuß wenigstens ein radialer Vorsprung oder Absatz ausgebildet, an dem die dem Plombenfuß zugewandte Seite mindestens einer Sperrlasche anliegt. Dieser radiale Vorsprung verhindert, dass die Sperrlasche versehentlich in die falsche Richtung verformt und die Sicherheitsplombe somit versehentlich entwertet wird. Wird die Sicherheitsplombe in die Aufnahme eines geöffneten Verschlusses eingesetzt und der Verschluss anschließend geschlossen, so wird die mindestens eine Sperrlasche zu dem Plombenfuß hin verformt. Diese Verformung würde zu einer plastischen Verformung in dem mindestens einen Scharnierelement und somit zu einer Entwertung der Plombe führen. Eine solche Plombe könnte man nicht mehr gebrauchen. Liegt aber die Sperrlasche in dieser Bewegungsrichtung an einem Vorsprung an, wird die plastische Verformung des Scharnierelements durch eine Bewegung in diese Richtung verhindert. Somit wird auch die fälschliche Entwertung der Plombe auf diese Weise verhindert.

Gemäß einer vorteilhaften Weiterentwicklung des ersten Aspekts der vorliegenden Erfindung sin die mindestens zwei Rastvorsprünge an dem Plombenfuß elastisch gelagert und stehen in radialer Richtung nach innen vor. In einem solchen Fall eignet sich keine Aussparung als Aufnahme sondern ein Vorsprung, beispielsweise ein pilzköpfiger Vorsprung. Die mindestens zwei elastischen Rastvorsprünge umgreifen den Pilzkopf dabei und bilden eine Hinterschneidung mit diesem. Vorteilhafter Weise sind mehr als zwei elastische Rastvorsprünge vorgesehen. Die Pilzkopfform ist aber nur ein Beispiel für einen Aufnahmevorsprung. Wichtig ist nur, dass der Vorsprung mindestens eine Fläche Aufweist, die eine Hinterschneidung mit dem mindestens einen Rastvorsprung der Sicherheitsplombe ermöglicht. Ebenfalls nicht maßgeblich ist die Form der Oberfläche des Vorsprungs, welche der Sicherheitsplombe zugewandt ist. Vorzugsweise ist diese im Wesentlichen kreisförmig und etwa halbkugelig gewölbt. Sie kann aber auch eben ausgebildet sein und eine Kreisform mit einigen Einschnitten aufweisen oder zum Beispiel im Wesentlichen die Form eines Kreuzes.

Gemäß einer besonders vorteilhaften Weiterentwicklung des ersten Aspekts der vorliegenden Erfindung ist eine Mehrzahl von Rastvorsprüngen vorgesehen, die vorzugsweise in gleichen Winkelabständen zueinander um den Plombenfuß herum angeordnet sind, wobei weiter vorzugsweise eine gerade Anzahl von Rastvorsprüngen vorgesehen ist, insbesondere zwei, vier oder sechs Rastvorsprünge. Eine solche Ausbildung führt einerseits zu einer symmetrischen Sicherheitsplombe bzw. zu einem symmetrischen Plombenfuß, sodass der Nutzer weniger darauf achten muss, die Sicherheitsplombe in der richtigen Lage in die Aufnahme einzuführen. Die Mehrzahl von Rastvorsprüngen sorgt dabei für eine besonders leichte und gleichzeitige sichere und spielfreie Anbringung der Sicherheitsplombe an der entsprechenden Aufnahme.

Gemäß einer weiteren besonders vorteilhaften Weiterentwicklung des ersten Aspekts der vorliegenden Erfindung ist an dem Plombenkopf mindestens eine elastische Federzunge vorgesehen, die in radialer Richtung über den inneren Rand einer Sperrlasche hinaus hervorsteht. Wenn bei einer solchen Plombe die Sperrlasche aus ihrer Ausgangsstellung über die elastische Federzunge bewegt wird, hilft diese Federzunge anschließend dabei, ein Zurückkehren der Sperrlasche in die Ausgangsposition zu verhindern. Besonders vorteilhaft ausgebildet ist eine solche elastische Federlasche, wenn sie an ihrer dem Plombenfuß zugewandten Seite zumindest teilweise an dem Plombenkopf oder dem Plombenfuß anliegt. In diesem Fall weist die elastische Federlänge eine unterschiedliche effektive Biegelänge in die entgegen gesetzten Bewegungsrichtungen der zugehörigen Sperrlasche auf. Wird die Sperrlasche aus der Ausgangslage heraus verformt, biegt sie dabei die elastische Federlasche auf. Die Federlasche kann sich in diese Richtung frei verformen und übt nur einen relativ geringen Widerstand auf die Sperrlasche aus. Der Biegeradius der elastischen Federlasche in dieser Richtung ist relativ groß. In der Gegenrichtung liegt die elastische Federlasche an einem Teil der Sicherheitsplombe an. Dies verkürzt die effektive Länge der Federlasche für eine Biegung in dieser Richtung und verkleinert den Biegeradius der Federlasche. Dies hat zur Folge, dass die Federlasche in dieser Richtung wesentlich steifer als in der Gegenrichtung ist und die Federlasche somit einen wesentlich größeren Widerstand auf die Sperrlasche ausübt. Bei geeigneter Wahl der Geometrie der elastischen Federlasche kann der Rückkehrwiderstand, den sie auf die zugehörige Sperrlasche ausübt, so groß werden, dass ein Zurückkehren der Sperrlasche in die Ausgangsposition im Wesentlichen ausgeschlossen ist.

Gemäß einer vorteilhaften Weiterentwicklung des ersten Aspekts der vorliegenden Erfindung ist die mindestens eine elastische Federzunge an der Oberseite des Plombenkopfs und vorzugsweise zentral angebracht. Auf diese Weise kann die effektive Federlänge der elastischen Federlasche für ein Auslenken der Sperrlasche vom Plombenfuß weg besonders groß gemacht werden. Gleichzeitig kann diese Federlasche über den gesamten Radius des Plombenkopfs an diesem anliegen, wodurch die effektive Federlänge für eine Verformung zum Plombenfuß hin klein bleibt, da hierfür nur die Länge vom äußeren Rand des Plombenkopfs, bis zu dem die Federlasche an dem Plombenkopf anliegt, und dem freien Ende der Federlasche maßgeblich ist. Denkbar ist auch, dass eine Federlasche in dem Bereich des gegenüber liegenden Randes des Plombenkopfs an dem Plombenkopf angebracht ist, über den sie hervor steht.

Gemäß einer vorteilhaften Weiterentwicklung des ersten Aspekts der vorliegenden Erfindung ist an der Mantelfläche des Plombenkopfs mindestens ein radial vorstehender zweiter Rastvorsprung vorgesehen und an mindestens einer Sperrlasche eine Rastnase so angeordnet, dass sie dem zweiten Rastvorsprung radial gegenüber liegt und in axialer Richtung im Vergleich zu dem zweiten Rastvorsprung weiter zum Plombenfuß hin angeordnet ist. Gemäß dieser Ausbildung gleitet die Rastnase bei einer Verformung bzw. Bewegung der Sperrlasche aus ihrer Ausgangsposition heraus über den zweiten Rastvorsprung, wobei die Rastnase von dem zweiten Rastvorsprung radial nach außen gedrängt wird und/oder der zweite Rastvorsprung durch die Rastnase radial nach innen gedrängt wird. Erleichtert wird dies, wenn die entsprechenden Gleitflächen glatt ausgebildet sind und keinen zu stumpfen Winkel gegenüber der Längsachse der Sicherheitsplombe einnehmen. Wenn die Rastnase nun an dem zweiten Rastvorsprung vorbei geglitten ist, rastet diese mit dem zweiten Rastvorsprung ein. Dies verhindert zuverlässig ein Zurückkehren der Sperrlasche in ihre Ausgangsposition.

Gemäß einer vorteilhaften Weiterentwicklung des ersten Aspekts der vorliegenden Erfindung ist wenigstens die Rastnase oder der zweite Rastvorsprung elastisch federnd ausgebildet. Auf diese Weise kann die Rastnase mit geringem Kraftaufwand an dem zweiten Rastvorsprung vorbei gleiten. Bei nicht federnder Rastnase und zweitem Rastvorsprung kann die erforderliche radiale Verschiebung aber auch durch das mindestens eine Scharnierelement bereit gestellt werden, das sich dementsprechend elastisch verlängert.

Gemäß einem zweiten Aspekt der vorliegenden Erfindung weist ein Sicherheitsverschluss ein erstes Verschlussteil und ein zweites Verschlussteil auf, welches zwischen einer Offenstellung und einer Schließstellung relativ zu dem ersten Verschlussteil hin und her bewegbar ist. Dabei weist das erste Verschlussteil eine Aufnahme auf, an welcher der Plombenfuß einer Sicherheitsplombe nach dem vorangehenden Aspekt und seinen vorteilhaften Weiterentwicklungen so einrastbar ist, dass sich, wenn sich das zweite Verschlussteil in der Schließstellung befindet, mindestens eine Sperrlasche der Sicherheitsplombe mit dem zweiten Verschlussteil auf der dem ersten Verschlussteil entgegen gesetzten Seite des zweiten Verschlussteils zumindest teilweise überlappt.

Auf diese Weise kann ein Sicherheitsverschluss bereit gestellt werden, bei dem sicher angezeigt wird, ob der Verschluss bereits einmal geöffnet wurde, wenn eine Sicherheitsplombe in die entsprechende Aufnahme eingesetzt ist. Durch die Überlappung der Sperrlasche der Sicherheitsplombe mit dem zweiten Verschlussteil kann dieses nicht von dem ersten Verschlussteil weg bewegt werden, ohne die Sperrlasche aus ihrer Ausgangsposition heraus zu bewegen und zu verformen. Bei einer Bewegung des zweiten Verschlussteils relativ zu dem ersten Verschlussteil innerhalb der Verschlussebene weist die Sperrlasche der Sicherheitsplombe einen Vorsprung in Richtung Plombenfuß auf, der in eine Vertiefung oder ein Durchgangsloch in dem zweiten Verschlussteil eingreift.

Gemäß einer vorteilhaften Weiterentwicklung des zweiten Aspekts der vorliegenden Erfindung weist das zweite Verschlussteil eine Aussparung und/oder ein Durchgangsloch auf und der Plombenfuß ist dort hindurch führbar und in der Schließstellung des zweiten Verschlussteils in der Plombenfußaufnahme des ersten Verschlussteils einrastbar. Diese Ausbildung ist besonders vorteilhaft, da sich auf diese Weise besonders leicht ein Formschluss zwischen Sicherheitsplombe und zweitem Verschlussteil oder beiden Verschlussteilen herstellen lässt, sodass die Sicherheitsplombe bei geschlossenem Verschluss nicht innerhalb der Verschlussebene gedreht, gekippt und/oder verschoben werden kann, und die Sicherheitsplombe bei geöffnetem Verschluss innerhalb der Verschlussebene gedreht, gekippt und/oder verschoben werden kann, um die Sicherheitsplombe von der Aufnahme zu entfernen.

Gemäß einer vorteilhaften Weiterentwicklung des zweiten Aspekts der vorliegenden Erfindung ist der Plombenfuß der Sicherheitsplombe in der Schließstellung des zweiten Verschlussteils so an der Plombenfußaufnahme des ersten Verschlussteils einrastbar, dass sich die Sicherheitsplombe mit dem zweiten Verschlussteil derart formschlüssig in Anlage befindet, dass die Sicherheitsplombe gegenüber dem ersten Verschlussteil im Wesentlichen unbeweglich gehalten, insbesondere nicht drehbar, kippbar und/oder verschiebbar ist, wobei die Sicherheitsplombe in der Offenstellung des zweiten Verschlussteils gegenüber dem ersten Verschlussteil bewegbar, insbesondere drehbar, kippbar und/oder verschiebbar ist. Dieser Aspekt ist besonders vorteilhaft, da der Formschluss nur zwischen zweitem Verschlussteil und Sicherheitsplombe hergestellt wird, sodass die Gestaltung des ersten Verschlussteils freier erfolgen kann.

Gemäß einer vorteilhaften Weiterentwicklung des zweiten Aspekts der vorliegenden Erfindung weist die Aufnahme an dem ersten Verschlussteil mindestens eine Aussparung auf, vorzugsweise mit einem im Wesentlichen rotationssymmetrischen Abschnitt mit mindestens einer radialen Aufweitung nach außen, in die ein Plombenfuß einer Sicherheitsplombe einführbar ist. Auf diese Weise kann ein Verschluss für eine Sicherheitsplombe geschaffen werden, die durch Drehen der Plombe um ihre Längsachse von dem Verschluss gelöst werden kann. Im Montagezustand bildet dabei der rotationssymmetrische Abschnitt der Aufnahme die Rastverbindung mit den mindestens zwei Rastvorsprüngen der Sicherheitsplombe aus. Zum Lösen der Hinterschneidung werden die mindestens zwei Rastvorsprünge in die Position der mindestens zwei radialen Aufweitungen gedreht.

Gemäß einer vorteilhaften Weiterentwicklung des zweiten Aspekts der vorliegenden Erfindung weist die Aufnahme an dem ersten Verschlussteil eine im Wesentlichen kreisringförmige Aussparung mit mindestens zwei radialen Aufweitungen nach außen und/oder innen auf, in die die mindestens zwei Rastvorsprünge einführbar sind. Auf diese Weise ist die Plombenfußaufnahme besonders einfach und günstig herzustellen und gleichzeitig wenig anfällig für Beschädigungen. Hierbei ist zu beachten, dass die Verschlusselemente nicht immer sehr pfleglich behandelt werden und beispielsweise bei einem Einsatz an Sterilisiercontainern auch den Sterilisiervorgang schadlos überstehen müssen.

Gemäß einer anderen vorteilhaften Weiterentwicklung des zweiten Aspekts der vorliegenden Erfindung ist die Aufnahme an dem ersten Verschlussteil ein pilzförmiger Vorsprung, auf den ein Plombenfuß einer Sicherheitsplombe aufsteckbar ist. Eine solche Aufnahme ermöglicht ein Aufstecken der Sicherheitsplombe auf den pilzförmigen Vorsprung von oben und ein Lösen derselben von dem Vorsprung durch Verkippen der Sicherheitsplombe gegenüber dem Vorsprung und anschließendes Abziehen der Plombe von dem Vorsprung. Dabei wird beim Verkippen der Plombe gegenüber dem Vorsprung mindestens ein elastischer Rastvorsprung nach außen gebogen, was durch eine günstige Hebelwirkung vereinfacht wird.

Gemäß einer vorteilhaften Weiterentwicklung des zweiten Aspekts der vorliegenden Erfindung verfügt der Sicherheitsverschluss über einen zusätzlichen bekannten Schließmechanismus verfügt, insbesondere über einen Rastmechanismus, mit dem das zweite Verschlussteil an dem ersten Verschlussteil arretierbar ist. Auf diese Weise ist der Sicherheitsverschluss nicht für das eigentliche Verschließen und/oder Verriegeln des ersten und zweiten Verschlussteils zuständig sondern dient lediglich dazu, den Sicherheitsaspekt des Sicherheitsverschlusses zu erfüllen. Das heißt, der Sicherheitsverschluss ist dafür da, sicher anzuzeigen, dass er nach dem Anbringen der Sicherheitsplombe noch nicht geöffnet wurde, d.h. noch immer geschlossen ist. Um einen Austausch der Sicherheitsplomben zu erkennen, können diese individualisiert sein, beispielsweise durch fortlaufende Nummerierung oder durch eine Unterschrift, Signierung, etc. derselben.

Gemäß einem dritten Aspekt der vorliegenden Erfindung weist ein Sicherheitsbehälter eine Behälterwanne, einen Deckel und einen Sicherheitsverschluss nach dem zweiten Aspekt der vorliegenden Erfindung und seinen vorteilhaften Ausgestaltungen auf, wobei eines von dem ersten und dem zweiten Verschlussteil jeweils an einem von der Behälterwanne und dem Deckel vorgesehen ist. Auf diese Weise kann beispielsweise ein Behälter realisiert werden, dessen Deckel an einer Seite der Behälterwanne angelenkt ist. In diesem Fall reicht ein Sicherheitsverschluss aus, der an einer anderen Seite, vorzugsweise der gegenüber liegenden Seite, der Behälterwanne angeordnet ist, an der der Deckel angelenkt ist. Es kann aber auch ein Behälter realisiert werden, bei dem der Deckel nicht an der Behälterwanne angelenkt ist. Um einen zuverlässigen Verschluss zu gewährleisten, sollten vorzugsweise zwei Sicherheitsverschlüsse an gegenüber liegenden Seiten des Behälters vorgesehen sein.

Gemäß einer vorteilhaften Weiterentwicklung des dritten Aspekts der vorliegenden Erfindung ist das erste Verschlussteil oder das zweite Verschlussteil an einer Verschlusslasche vorgesehen ist, welche entsprechend an der Behälterwanne und/oder dem Deckel schwenkbar vorgesehen ist. Auf diese Weise kann ein Verschluss bereit gestellt werden, bei dem das zweite Verschlussteil gegenüber dem ersten Verschlussteil verschenkt wird. Diese Ausbildung ermöglicht einen besonders einfachen Aufbau des Sicherheitsverschlusses und der Sicherheitsplombe bei gleichzeitig sehr hohem Sicherheitsniveau. Vorteilhafter Weise weist eine schräge Verschlusslasche auf einen nicht ordnungsgemäßen Schließzustand hin und bei einem korrekten Schließzustand des Sicherheitsverschlusses ist die Verschlusslasche beispielsweise vertikal oder horizontal positioniert. Eine Schrägstellung einer Verschlusslasche ist auch für einen Nutzer mit geringer Aufmerksamkeit leicht zu erkennen, wodurch das Sicherheitsniveau des Sicherheitsbehälters angehoben wird.

Gemäß einem vierten Aspekt der vorliegenden Erfindung weist ein Sterilisiercontainer eine Containerwanne mit mindestens einem ersten Verschlussteil auf, welches eine Aussparung aufweist mit zwei gegenüberliegenden kreisbogenförmigen Anlageflächen, die an zwei Kreisbogenabschnitten ausgebildet sind, zwischen denen zwei radiale Aufweitungen vorgesehen sind. Diese Aussparung ist daran angepasst, einen Plombenfuß einer Sicherheitsplombe, welcher einen im Wesentlichen kreisförmigen Querschnitt und zwei radial nach außen vorstehende Rastvorsprünge aufweist und in axialer Richtung in die Aussparung einschiebbar ist, so aufzunehmen, dass die Rastvorsprünge eine rastende Hinterschneidung mit den Kreisbogenabschnitten herstellen und eine Mantelfläche des Plombenfußes teilweise in Anlage an die Anlageflächen gelangt. Darüber hinaus ist die Aussparung daran angepasst, dass die Rastverbindung zwischen den Rastvorsprüngen und den Kreisbogenabschnitten durch eine Drehung der Sicherheitsplombe um ihre Längsachse um im Wesentlichen 90° aufhebbar ist, sodass die Sicherheitsplombe aus der Aussparung entnehmbar ist. Der Sterilisiercontainer weist zudem einen Containerdeckel auf, der über mindestens eine Verschlusslasche verfügt, welche schwenkbar an ihm angebracht ist, sodass sie von einer Offenstellung in eine Schließstellung hin und her bewegbar ist, und einen zweiten Verschlussteil aufweist, welcher ein Durchgangsloch aufweist, das einen im Wesentlichen nicht rotationssymmetrischen Querschnitt aufweist und daran angepasst ist, einen Plombenkopf der Sicherheitsplombe in Drehrichtung formschlüssig so aufzunehmen, dass eine Bewegung der Verschlusslasche relativ zu der Sicherheitsplombe in Richtung zu der Offenstellung hin zu einer Anlage zumindest eines Teils der Umrandung des Durchgangslochs mit mindestens einer an dem Plombenkopf der Sicherheitsplombe vorgesehenen Sperrlasche führt und eine weitergehende Bewegung der Verschlusslasche in dieser Richtung zu einer zumindest teilweisen plastischen Verformung der mindestens einen Sperrlasche führt, sodass der das Durchgangsloch der Verschlusslasche über den Plombenkopf hinweg führbar ist.

Auf diese Weise kann ein Sterilisiercontainer bereit gestellt werden, bei dem durch eine Sicherheitsplombe gewährleistet werden kann, dass der Sterilisierbehälter nach der Sterilisation nicht geöffnet wurde, solange die Sicherheitsplombe intakt ist. Dies kann auch mit geringer Aufmerksamkeit auch noch leicht und sicher bestimmt werden.

Gemäß einer vorteilhaften Weiterentwicklung des vierten Aspekts der vorliegenden Erfindung ist zwischen der Verschlusslasche und dem ersten Verschlussteil ein zusätzlicher Verriegelungs- bzw. Verschlussmechanismus vorgesehen ist, sodass der Sicherheitsverschluss in der Schließstellung der Verschlusslasche im Wesentlichen kraftfrei ist. Auf diese Weise dient der Sicherheitsverschluss nur der Gewährleistung des Schließzustandes seit dem Einsetzen der Sicherheitsplombe. Der eigentliche Verschluss wird durch weitere Verschlussbauteile erzielt, die an einem gesonderten Verschluss oder an dem Sicherheitsverschluss vorgesehen sind. Wenn der Sicherheitsverschluss und damit die Sicherheitsplombe kraftfrei gehalten werden, kann die Sicherheitsplombe nicht beschädigt werden, wenn der Sterilisiercontainer beispielsweise am Containerdeckel angehoben wird.

Weitere Vorteile und Merkmale der Erfindung sind dem Fachmann aus den beigefügten Figuren und der detaillierten Beschreibung der Ausführungsbeispiele ersichtlich.
- Fig. 1: zeigt eine Seitenansicht einer Sicherheitsplombe gemäß einem ersten, nicht zur Erfindung gehörenden, Ausführungsbeispiel im nicht entwerteten Zustand;
- Fig. 2: zeigt eine perspektivische Ansicht einer Sicherheitsplombe gemäß dem ersten, nicht zur Erfindung gehörenden, Ausführungsbeispiel im nicht entwerteten Zustand;
- Fig. 3: zeigt eine perspektivische Ansicht einer Sicherheitsplombe gemäß dem ersten, nicht zur Erfindung gehörenden, Ausführungsbeispiel im entwerteten Zustand;
- Fig. 4: zeigt eine weitere perspektivische Ansicht einer Sicherheitsplombe gemäß dem ersten, nicht zur Erfindung gehörenden, Ausführungsbeispiel im nicht entwerteten Zustand;
- Fig. 5: zeigt eine perspektivische Ansicht einer Aufnahme für eine Sicherheitsplombe gemäß einem Ausführungsbeispiel der vorliegenden Erfindung;
- Fig. 6A: zeigt eine perspektivische Ansicht eines Sicherheitsverschlusses gemäß einem Ausführungsbeispiel der vorliegenden Erfindung;
- Fig. 6B: zeigt eine perspektivische Ansicht des Sicherheitsverschlusses gemäß dem ersten Ausführungsbeispiel der vorliegenden Erfindung mit der Sicherheitsplombe;
- Fig. 6C: zeigt eine perspektivische Ansicht eines Teils des Sicherheitsverschlusses gemäß dem ersten Ausführungsbeispiel der vorliegenden Erfindung mit der entwerteten Sicherheitsplombe;
- Fig. 7A: zeigt eine isometrische Ansicht eines Sterilisiercontainers mit einem Sicherheitsverschluss und einer Sicherheitsplombe gemäß dem ersten Ausführungsbeispiel der vorliegenden Erfindung;
- Fig. 7B: zeigt einen vergrößerten Ausschnitt von Fig. 7A;
- Fig. 8: zeigt eine Seitenansicht einer Sicherheitsplombe gemäß einem zweiten Ausführungsbeispiel der vorliegenden Erfindung im nicht entwerteten Zustand;
- Fig. 9: zeigt eine perspektivische Ansicht einer Sicherheitsplombe gemäß dem zweiten Ausführungsbeispiel der vorliegenden Erfindung im nicht entwerteten Zustand;
- Fig. 10: zeigt eine weitere perspektivische Ansicht einer Sicherheitsplombe gemäß dem zweiten Ausführungsbeispiel der vorliegenden Erfindung im nicht entwerteten Zustand;
- Fig. 11: zeigt eine Ansicht einer Sicherheitsplombe gemäß einem zweiten Ausführungsbeispiel der vorliegenden Erfindung im nicht entwerteten Zustand von oben;
- Fig. 12: zeigt eine Aufnahme eines Sicherheitsverschlusses für eine Sicherheitsplombe gemäß dem zweiten Ausführungsbeispiel der vorliegenden Erfindung;
- Fig. 13: zeigt eine Seitenansicht einer Sicherheitsplombe und einer Aufnahme eines Sicherheitsverschlusses gemäß dem zweiten Ausführungsbeispiel der vorliegenden Erfindung;
- Fig. 14A: zeigt eine perspektivische Ansicht eines Sterilisiercontainers mit einem Sicherheitsverschluss und der Sicherheitsplombe gemäß dem zweiten Ausführungsbeispiel der vorliegenden Erfindung;
- Fig. 14B: zeigt einen vergrößerten Ausschnitt von Fig. 13A;
- Fig. 15: zeigt eine perspektivische Ansicht einer Sicherheitsplombe gemäß einem dritten Ausführungsbeispiel der vorliegenden Erfindung im nicht entwerteten Zustand;
- Fig. 16: zeigt eine weitere perspektivische Ansicht einer Sicherheitsplombe gemäß dem dritten Ausführungsbeispiel der vorliegenden Erfindung im nicht entwerteten Zustand;
- Fig. 17: zeigt eine perspektivische Ansicht einer Sicherheitsplombe gemäß einem vierten Ausführungsbeispiel der vorliegenden Erfindung im nicht entwerteten Zustand; und
- Fig. 18: zeigt eine weitere perspektivische Ansicht einer Sicherheitsplombe gemäß dem vierten Ausführungsbeispiel der vorliegenden Erfindung im nicht entwerteten Zustand.

Im Folgenden sind ein nicht zur Erfindung gehörendes Ausführungsbeispiel sowie mehrere Ausführungsbeispiele der vorliegenden Erfindung unter Bezugnahme auf die Figuren im Detail beschrieben.

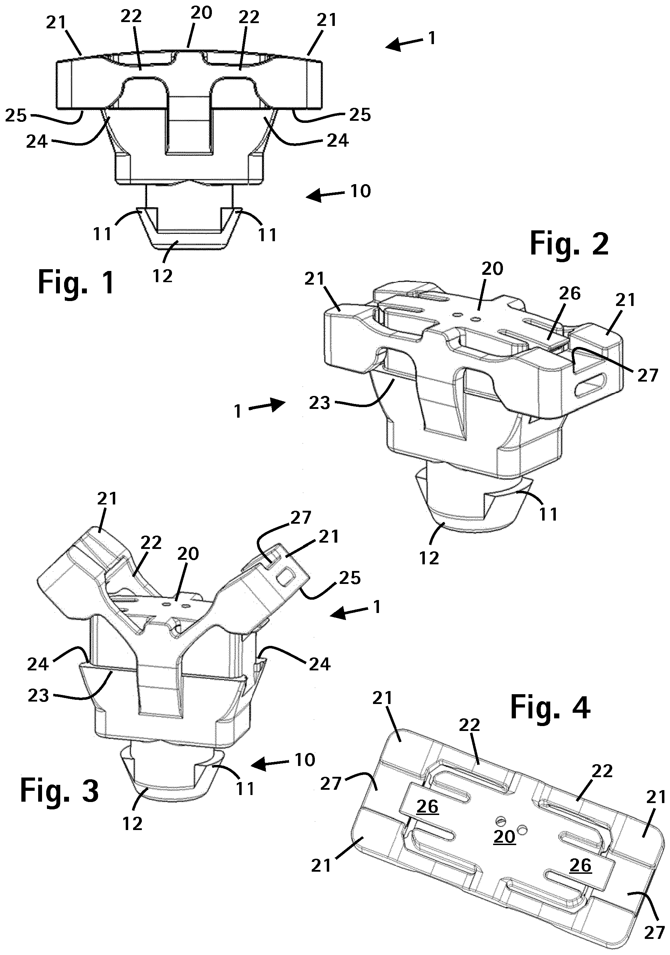

Ein erstes Ausführungsbeispiel, welches nicht zu der Erfindung gehört, ist im Folgenden unter Bezugnahme auf die Fig. 1 bis 7 beschrieben.

Die Sicherheitsplombe 1 gemäß dem ersten, nicht zur Erfindung gehörenden, Ausführungsbeispiel verfügt über einen Plombenfuß 10 mit zwei Rastvorsprüngen 11 und einen Plombenkopf 20. Darüber hinaus weist die Sicherheitsplombe 1 zwei Sperrlaschen 21 auf, wobei beide Sperrlaschen 21 jeweils über zwei Scharnierelemente 22 (nur jeweils eines ist in Fig. 1 gezeigt) mit dem Plombenkopf 20 bewegbar verbunden sind. Die gesamte Sicherheitsplombe 1 ist durch ein Spritzgussverfahren mit Zwangsentformung einstückig aus Kunststoff hergestellt. Die Scharnierelemente 22 sind plastisch verformbar, das heißt bei einer Biegung der Scharnierelemente 22 über ein bestimmtes Maß hinaus tritt ein so genannter Weißbruch in den Scharnierelementen 22 ein. Ab welcher Biegung bzw. Verformung der Weißbruch und somit die plastische Verformung der Scharnierelemente 22 eintritt, hängt von der Höhe und Breite der Scharnierelemente 22 sowie von ihren Materialeigenschaften ab, bei diesem Ausführungsbeispiel also von dem gewählten Kunststoff.

Wie dies aus den Fig. 1 bis 4 ersichtlich ist, sind die beiden Sperrlaschen 21 bei diesem nicht zur Erfindung gehörenden Ausführungsbeispiel in radialer Richtung gegenüber liegend ausgebildet, bei diesem Ausführungsbeispiel ist die gesamte Sicherheitsplombe 1 sogar spiegelsymmetrisch ausgebildet, was allgemein eine vorteilhafte Ausgestaltung für eine erfindungsgemäße Sicherheitsplombe 1 ist. Die Scharnierelemente 22, mit denen die Sperrlaschen 21 am Plombenkopf 20 gehalten sind, sind seitlich an dem Plombenkopf vorgesehen. Der Plombenfuß 10 weist einen im Wesentlichen kreisförmigen Querschnitt auf. Bei diesem Ausführungsbeispiel erstreckt sich von dem Plombenkopf 20 ein zylinderförmiger Vorsprung 12, an dem die beiden Rastvorsprünge 11 radial gegenüberliegend nach außen vorstehend vorgesehen sind. Die Rastvorsprünge 11 stehen in denselben Richtungen gegenüber dem Plombenfuß 10 vor, wie die Sperrlaschen 21 gegenüber dem Plombenkopf 20 vorstehen.

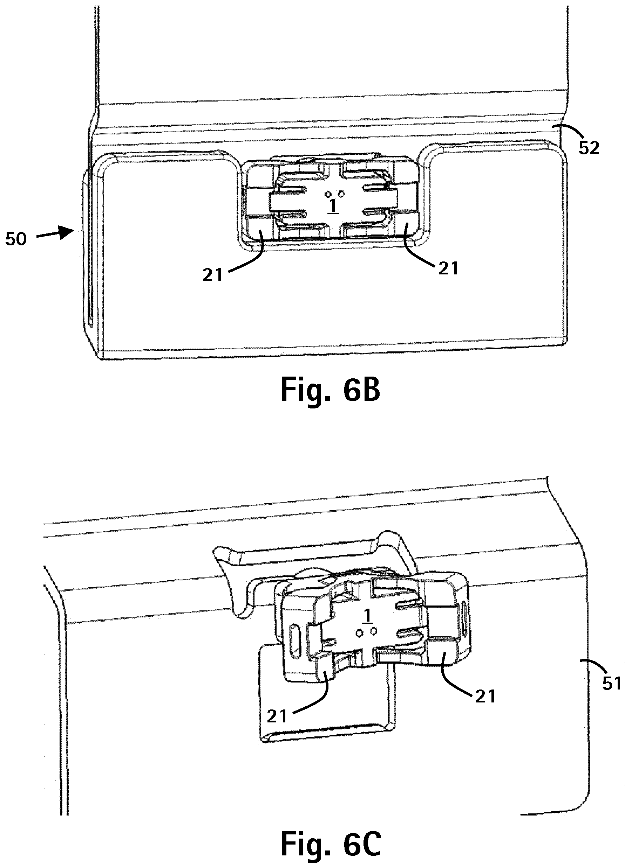

Der Plombenkopf 20 weist einen im Wesentlichen rechteckigen Querschnitt auf und die beiden Sperrlaschen 21 und die zugehörigen Scharnierelemente 22 ergeben ebenfalls einen im Wesentlichen rechteckigen Querschnitt, wie dies insbesondere aus Fig. 4 und Fig. 6B ersichtlich ist. Außerdem steht jede der beiden Sperrlaschen 21 radial, d.h. bei diesem Ausführungsbeispiel seitlich, gegenüber dem Plombenfuß 10 und dem Plombenkopf 20 hervor.

Am Plombenkopf 20 sind zudem zwei Vorsprünge 23 ausgebildet, die seitlich von dem Plombenkopf 20 vorstehen. Diese beiden Vorsprünge 23 erstrecken sich entlang der längeren Seite des im Wesentlichen rechteckigen Plombenkopfs 20 und bilden insgesamt vier Stützflächen 24, an denen jeweils die dem Plombenfuß 10 zugewandte Seite 25 der beiden Sperrlaschen 21 anliegen.

Wie dies in der Fig. 4 gezeigt ist, weist die Sicherheitsplombe 1 gemäß diesem Ausführungsbeispiel zwei elastische Federzungen 26 auf. Diese elastischen Federzungen 26 sind zentral an der Oberseite des Plombenkopfs 20 vorgesehen bzw. ausgebildet und stehen von dem Plombenkopf 20 in radialer Richtung über den inneren Rand der jeweiligen Sperrlasche 21 hinaus hervor. Dies bedeutet, dass das freie distale Ende jeder elastischen Federlasche 26 einen Teil der Oberseite, d.h. der dem Plombenfuß 10 abgewandten Seite, jeweils einer Sperrlasche 21 überlappt. In diesen Überlappungsbereichen weisen die beiden Sperrlaschen 21 zudem Vertiefungen 27 auf, sodass die Oberseiten der elastischen Federzungen 26, die Oberseite des Plombenkopfs 20 und die Oberseite der beiden Sperrlaschen 21 im Wesentlichen in einer Fläche liegen. Dies führt zu einer besonders angenehmen Haptik der Sicherheitsplombe 1 beim Einsetzen derselben in eine entsprechende Plombenfußaufnahme sowie zu einem harmonischen Erscheinungsbild der Sicherheitsplombe 1.

Die beiden elastischen Federzungen 26 sind im Übrigen nur mit ihren proximalen Enden im zentralen Bereich des Plombenkopfs 20 mit demselben verbunden. Die Unterseite der elastischen Federlaschen 26, also die dem Plombenfuß 10 zugewandten Seiten der Federlaschen 26 sind nicht mit dem Plombenkopf 20 verbunden. Diese Unterseiten liegen entweder an dem Plombenkopf 20 an, oder es ist sogar ein Spalt zwischen der Federlasche 26 und dem Plombenkopf 20 ausgebildet. Auf diese Weise können die elastischen Federlaschen 26 zwar leicht von dem Plombenkopf 20 nach oben, also von dem Plombenfuß 10 weg, gebogen werden, um die Sperrlaschen 21 passieren zu lassen, andererseits können diese elastischen Federlaschen 26 nur schwer in die Gegenrichtung gebogen werden, um die Sperrlaschen 21 in der entgegen gesetzten Richtung passieren zu lassen, nämlich bei dem Versuch in ihre Ausgangsposition zurück zu kehren. Dies liegt daran, dass die Unterseiten der Federlaschen 26 durch einen Teil der Oberseite des Plombenkopfs 20 gestützt werden, sodass in die elastischen Federlaschen 26 beiden vorstehend beschriebenen Biegerichtungen eine unterschiedliche effektive Länge aufweisen.

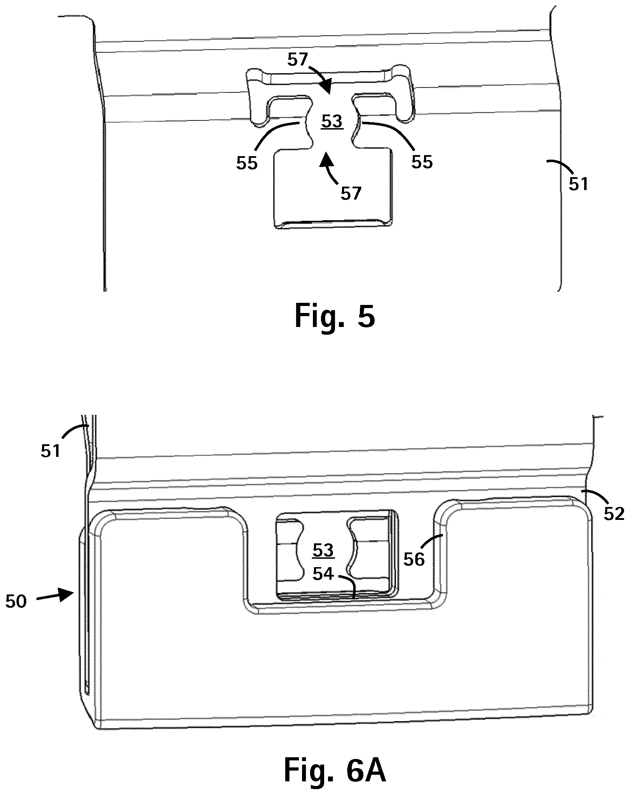

In den Fig. 5 und 6A bis 6C ist ein Sicherheitsverschluss 50 gemäß einem Ausführungsbeispiel mit einem ersten Verschlussteil 51 und einem zweiten Verschlussteil 52 gezeigt. Dieser Sicherheitsverschluss 50 ist daran angepasst, gemeinsam mit der Sicherheitsplombe 1 gemäß dem ersten, dritten oder vierten Ausführungsbeispiel verwendet zu werden, wobei das erste Ausführungsbeispiel nicht zur Erfindung gehört. Die Sicherheitsplombe 1 gemäß dem ersten, dritten oder vierten Ausführungsbeispiel und der Sicherheitsverschluss 50 gemäß dem ersten Ausführungsbeispiel wirken zusammen wie ein Schlüssel und ein zugehöriges Schloss oder ein Stecker und die zugehörige Buchse.

Bei dem Sicherheitsverschluss50 gemäß diesem Ausführungsbeispiel ist das zweite Verschlussteil 52 zwischen einer Offenstellung und einer Schließstellung relativ zu dem ersten Verschlussteil 51 hin und her bewegbar, indem es diesem gegenüber schwenkbar ist. Das erste Verschlussteil 51 weist eine Aufnahme 53 auf, an der der Plombenfuß 10 der Sicherheitsplombe 1 einrastbar ist. Die Sicherheitsplombe 1 ist insbesondere auch dann an der Aufnahme 53 einrastbar, wenn sich das zweite Verschlussteil 52 in der Schließstellung befindet, d.h. wenn das zweite Verschlussteil 52 das erste Verschlussteil 51 überdeckt.

Die Aufnahme 53 ist so gestaltet, dass sie Bereiche aufweist, die Hinterschneidungen mit den Rastvorsprüngen 11 des Plombenfußes 10 der Sicherheitsplombe 1 herstellen können. Bei diesem Ausführungsbeispiel sind dies die beiden bogenförmigen Abschnitte 55, welche die Aufnahme seitlich begrenzen. Zwischen den beiden bogenförmigen bzw. kreisbogenförmigen Abschnitten 55 sind zwei offene Abschnitte 57 ausgebildet. Diese offenen Abschnitte 57 sind so ausgebildet, dass sie keine Hinterschneidungen mit den Rastvorsprüngen 11 der Sicherheitsplombe 1 ausbilden können. Soll die Sicherheitsplombe 1 aus der Aufnahme 53 entfernt werden, muss die Sicherheitsplombe relativ zu der Aufnahme 53 so weit um ihre Achse gedreht werden, dass sich die Rastvorsprünge 11 der Sicherheitsplombe 1 in den freien Abschnitten 57 befinden. In diesem Zustand kann die Sicherheitsplombe 1 aus der Aufnahme 53 gezogen werden.

Wenn sich das zweite Verschlussteil 52 in der Schließstellung befindet und eine Sicherheitsplombe 1 gemäß dem ersten Ausführungsbeispiel in die Aufnahme 52 des ersten Verschlussteils51 eingebracht wird, überdecken die beiden Sperrlaschen 21 der Sicherheitsplombe 1 einen Teil des zweiten Verschlussteils 52, wie dies in der Fig. 6B gezeigt ist. Damit das zweite Verschlussteil 52 im Verschlusszustand kein Spiel gegenüber dem ersten Verschlussteil 51 hat, kann die Sicherheitsplombe 1 so bemessen sein, dass die Unterseite der Sperrlaschen 21, also die dem Plombenfuß 10 zugewandten Seiten der Sperrlaschen 21, an der Oberfläche des zweiten Verschlussteils 52 anliegen.

Bei dem vorliegenden Ausführungsbeispiel weist das zweite Verschlussteil 52 ein Durchgangsloch 54 auf, durch welches der Plombenfuß 10 der Sicherheitsplombe 1 hindurch geführt werden kann und in der Aufnahme bzw. Plombenfußaufnahme 53 des ersten Verschlussteils 51 einrastbar ist, wenn sich das zweite Verschlussteil 52 in der Schließstellung des Sicherheitsverschlusses 50 befindet. Wenn sich der Sicherheitsverschluss 50 in der Schließstellung befindet und eine Sicherheitsplombe 1 in die Aufnahme 53 in dem ersten Verschlussteil 51 des Sicherheitsverschlusses 50 eingebracht wird, bildet die Sicherheitsplombe 1 mit dem Verschlussteil 52 einen solchen Formschluss, dass die Sicherheitsplombe 1 nicht so weit gegenüber dem Sicherheitsverschluss 50 verdreht werden kann, dass die Sicherheitsplombe 1 von dem Sicherheitsverschluss 50 entfernt werden kann, wie dies vorstehend beschrieben ist, d.h. indem die Sicherheitsplombe 1 so weit gegenüber der Aufnahme 53 verdreht werden kann, dass sich die Rastvorsprünge 11 im Wesentlichen vollständig in den freien Abschnitten 57 befinden. Dies bedeutet, dass im Rahmen dieser Anmeldung der Begriff Formschluss nicht nur für einen solchen Formschluss steht, bei dem keinerlei Bewegung zwischen den betroffenen Bauteilen stattfinden kann, sondern dass die Bewegung lediglich nicht so groß sein darf, dass die Sicherheitsplombe 1 aus der Aufnahme 53 entnehmbar bzw. entfernbar ist.

Bei dem vorliegenden Ausführungsbeispiel ist an dem zweiten Verschlussteil 52 noch ein Verdickungsbauteil 56 vorgesehen. Dieses Verdickungsbauteil 56 erfüllt zwei Aufgaben. Zum Einen stellt es einen weiteren Formschluss (im vorstehend definierten Sinn) zwischen Sicherheitsplombe 1 und zweitem Verschlussteil 52 her, und zum Anderen schützt es die Sicherheitsplombe 1 gegen eine seitliche Krafteinwirkung. Das Verdickungsbauteil 56 schützt somit die Sicherheitsplombe 1 gegen eine unbeabsichtigte Entwertung bzw. eine Beschädigung. Mit dem Verdickungsbauteil 56 bestehen zwei Formschlüsse zwischen der Sicherheitsplombe 1 und dem Sicherheitsverschluss 50. Ein erster Formschluss liegt zwischen der Mantelfläche des Plombenkopfs 20 der Sicherheitsplombe 1 und dem inneren Rand des Durchgangslochs 54 vor und ein zweiter Formschluss liegt zwischen den seitlichen Flächen der beiden Sperrlaschen 21 und dem inneren Rand der Aussparung in dem Verdickungsbauteil 56 vor.

Bei diesem Ausführungsbeispiel dient der beschriebene Sicherheitsverschluss lediglich dazu, sicher zu stellen, dass der Sicherheitsverschluss 50 nicht geöffnet wurde, solange die Sicherheitsplombe 1 nicht entwertet ist, d.h. die Sperrlaschen gegenüber dem Plombenkopf verbogen sind. Ein Verriegeln des Verschlusses wird durch eine zusätzliche herkömmliche Verriegelungsvorrichtung erreicht, welche entweder zusätzlich an dem Sicherheitsverschluss 50 oder anderweitig zwischen den beiden Verschlussteilen vorgesehen ist und hier nicht weiter beschrieben ist.

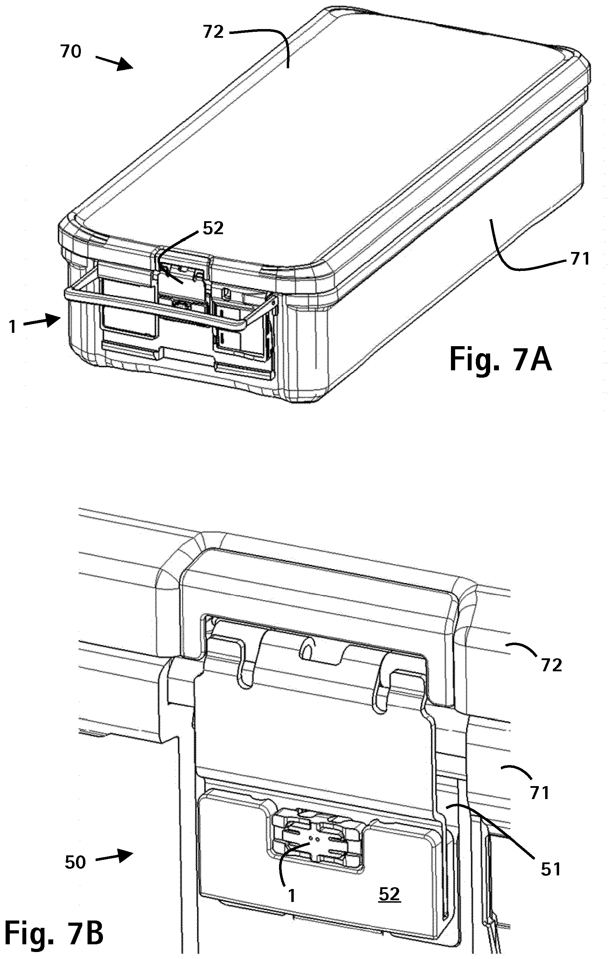

Der vorstehend beschriebene Sicherheitsverschluss kann beispielsweise an einem Sicherheitsbehälter 70 vorgesehen sein, der eine Behälterwanne 71 und einen Deckel 72 aufweist. Das erste Verschlussteil 51 ist dabei an der Behälterwanne 71 vorgesehen und das zweite Verschlussteil 52 ist an einer Verschlusslasche vorgesehen, welche gelenkig an dem Deckel 72 des Sicherheitsbehälters 70 angebracht ist. Ein Beispiel für solch einen Sicherheitsbehälter 70 ist ein medizinischer Sterilisiercontainer, wie er in der Fig. 7 exemplarisch gezeigt ist. Der Container gemäß diesem Ausführungsbeispiel ist im Wesentlichen symmetrisch ausgebildet, d.h. er weist je einen Sicherheitsverschluss an jeder seiner beiden Stirnseiten auf. Zum Öffnen des Containers 70, d.h. zum Abheben des Deckels 72 von der Containerwanne 71, werden die beiden Verschlusslaschen, die gelenkig an dem Deckel 72 befestigt sind, aufgeschwenkt, sodass jeweils das zweite Verschlussteil 52 von dem entsprechenden ersten Verschlussteil 51 schwenkend weg bewegt wird. Im Folgenden wird der Ablauf nur für eine Seite des Containers beschrieben, d.h. nur für einen Sicherheitsverschluss.

Dabei drängt der Randbereich des Durchgangslochs 54 in dem zweiten Verschlussteil 52 gegen die Unterseiten der beiden Sperrlaschen 21. Die Oberseiten der Sperrlaschen 21 drücken wiederum gegen die elastischen Federlaschen 26, sodass einerseits die Scharnierelemente 22 und andererseits die elastischen Federelemente 26 verbogen werden. Sind die elastischen Federelemente 26 hinreichend verbogen, können die Sperrlaschen 21 die freien Enden derselben passieren. Haben die Sperrlaschen die freien Enden der elastischen Federelemente 26 passiert, kehren diese in ihre Ausgangsposition zurück. Dazu sind die elastischen Federelemente 26 so bemessen, dass sie im Wesentlichen keine plastische Verformung erfahren. Die Sperrlaschen 21 werden ihrerseits so lange weiter aufgebogen, bis sie sich so weit aneinander angenähert haben, dass sie durch das Durchgangsloch 54 in dem zweiten Verschlussteil 52 passen. Anschließend kehren auch die Sperrlaschen 21 zu einem gewissen Grad in Richtung ihrer Ausgangsposition zurück. Ein vollständiges Zurückkehren der Sperrlaschen in ihre Ausgangsposition wird einerseits durch die plastische Verformung der Scharnierelemente 22 verhindert, welche während der Verbiegung eingetreten ist, und andererseits durch die elastischen Federelemente 26, die den Weg blockieren.

Der Sicherheitsverschluss 50 wurde auf diese Weise geöffnet und die Sicherheitsplombe 1 wurde gleichzeitig entwertet. Dabei ist die Sicherheitsplombe 1 nach wie vor einstückig, d.h. es wurden keine Teile von der Sicherheitsplombe 1 abgelöst oder getrennt. Die Entwertung der Sicherheitsplombe 1 ist durch die vorstehenden Sperrlaschen 21 sehr leicht wahrnehmbar. Jetzt ist auch ein Schließen des Sicherheitsverschlusses 50 nicht mehr möglich, ohne die Sicherheitsplombe 1 zu entfernen. Versucht man, den Sicherheitsverschluss 50 zu schließen, ohne die entwertete Sicherheitsplombe 1 vorher von dem Verschluss 50 zu entfernen, drückt der Randbereich des Durchgangslochs 54 in dem zweiten Verschlussteils 52 gegen die Sperrlaschen 21. Diese können aber nicht weit genug ausweichen, um es dem Durchgangsloch 54 zu ermöglichen, über die Sperrlaschen 21 zu rutschen. Einerseits verhindern dies die elastischen Federelemente 26, andererseits die Stützflächen 24. Der Container 70 kann also nicht mehr verschlossen werden.

Der Sicherheitsverschluss 50 und die Sicherheitsplombe 1 schützen aber auch vor einer mutwilligen Manipulation. Es ist vorstellbar, dass nach einem Öffnen des Verschlusses 50 jemand die beiden Sperrlaschen 21 zusammen drückt, d.h. an der Oberseite der Sicherheitsplombe 1 aufeinander zu bewegt, und so fixiert. Anschließend bewegt er das zweite Verschlussteil 52 über die Sperrlaschen 21 hinweg und hebt die Fixierung der Sperrlaschen 21 aneinander wieder auf. Diese Person kann zwar den Sicherheitsverschluss 50 mit großem Aufwand wieder schließen, er kann es aber nun nicht bewerkstelligen, die Sicherheitsplombe so aussehen zu lassen, als wäre sie niemals entwertet worden. Die beiden Sperrlaschen 21 stehen nach wie vor deutlich von der Sicherheitsplombe 1 vor. Sie können aufgrund der elastischen Federelemente 26 und der plastischen Verformung der Scharnierelemente 22 nicht mehr in ihre Ausgangsposition zurück gebracht werden.

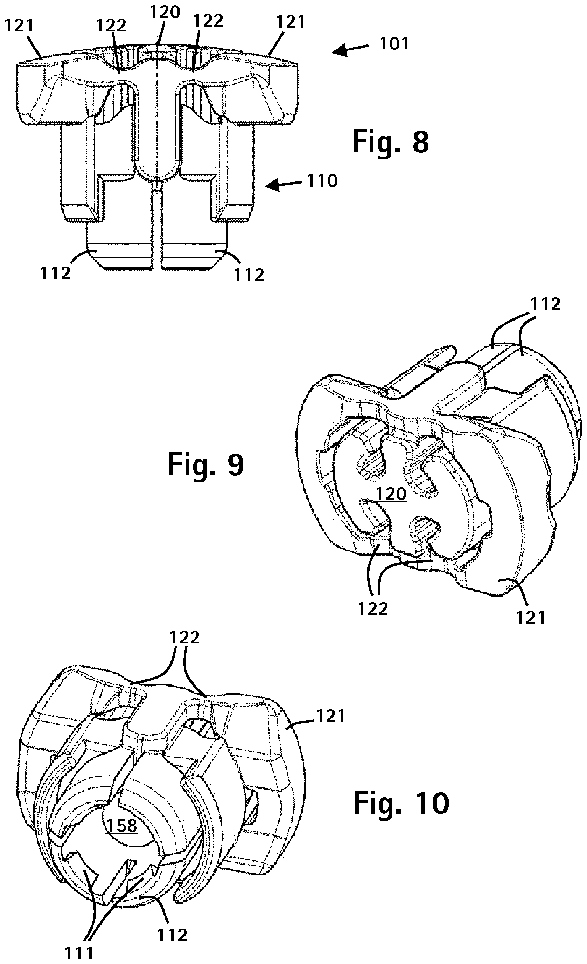

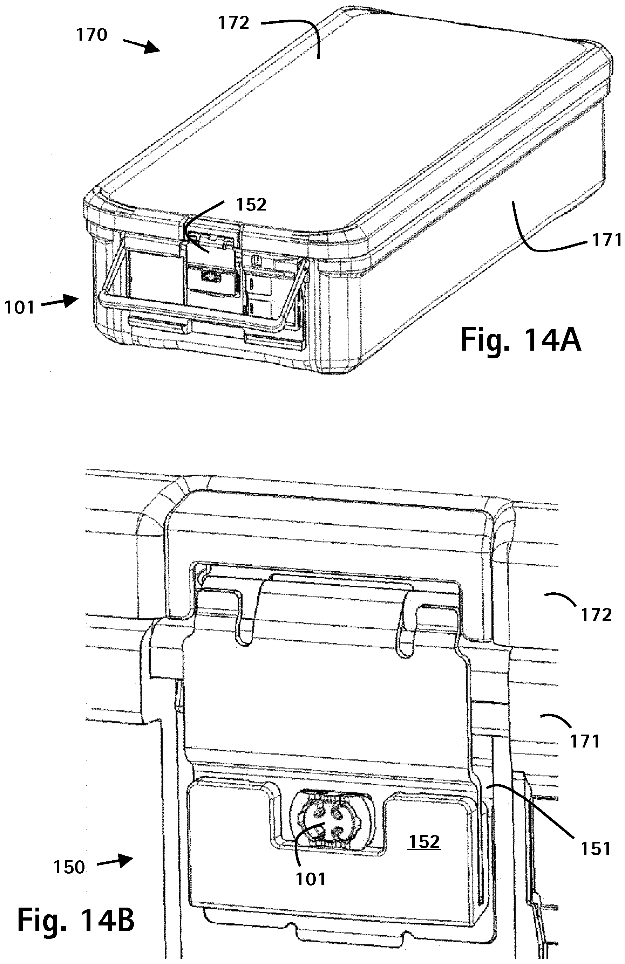

Im Folgenden ist ein zweites Ausführungsbeispiel der vorliegenden Erfindung unter Bezugnahme auf die Fig. 8 bis 14 beschrieben.

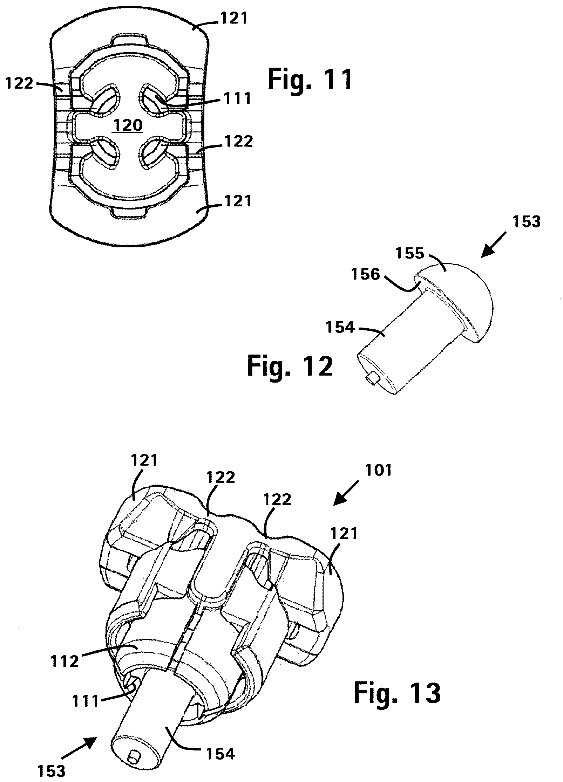

Die Sicherheitsplombe 101 gemäß dem zweiten Ausführungsbeispiel weist einen Plombenfuß 110 und einen Plombenkopf 120 auf. Zwei Sperrlaschen 121 sind an dem Plombenkopf 120 vergleichbar zu dem ersten Ausführungsbeispiel vorgesehen, allerdings ist der Querschnitt des Plombenkopfs 120 des zweiten Ausführungsbeispiels weniger rechtwinklig. Die beiden Sperrlaschen 121 sind mit je zwei Scharnierelementen 122 seitlich an dem Plombenkopf 120 angebracht und gegenüber diesem bewegbar. Der Plombenfuß 110 ist bei diesem Ausführungsbeispiel mit vier Federzungen 112 ausbildet, von denen je ein Rastvorsprung 111 radial nach innen vorsteht. Die vier Federzungen 112 ergeben zusammen einen im Wesentlichen kreisringförmigen Querschnitt und sind gleichmäßig um die Achse der Sicherheitsplombe 110 herum verteilt. Auch bei diesem Ausführungsbeispiel ist die Sicherheitsplombe 101 einstückig und aus Kunststoff ausgebildet. Allerdings wird diese Sicherheitsplombe 101 mit einem Spritzgussverfahren hergestellt, welches ohne Zwangsentformung auskommt. Dies wird dadurch erreicht, dass in axialer Richtung der Sicherheitsplombe 101 gesehen dort, wo die nach innen vorstehenden Rastvorsprünge 111 an den Federzungen 112 des Plombenfußes 110 ausgebildet sind, Aussparungen in dem Plombenkopf 120 vorgesehen sind, sodass die entsprechenden Formteile entnommen werden können (Fig. 11). Auch verfügt die Sicherheitsplombe gemäß dem zweiten Ausführungsbeispiel weder über elastische Federelemente 26 noch über Stützflächen 24.

Bei dieser Sicherheitsplombe 101 ist es nicht erforderlich, dass eine Art Formschluss gegenüber einer Drehung der Sicherheitsplombe 101 um ihre Längsachse zwischen ihr und einem Teil des Sicherheitsverschlusses 150 ausgebildet werden kann. Dies liegt daran, dass diese Sicherheitsplombe 101 an eine besondere Aufnahme 153 angepasst ist.

Die Aufnahme 153 weist, wie dies in Fig. 11 gezeigt ist, eine Pilzkopfform auf, d.h. sie hat einen Stiel 154 und einen Pilzkopf 155 und bildet so eine Hinterschneidungsfläche 156 aus. Der Stiel ist an einem der beiden Verschlussteile 151, 152 des Sicherheitsverschlusses 150 angebracht. Wird die Sicherheitsplombe 101 gemäß dem zweiten Ausführungsbeispiel auf die Aufnahme 153 aufgesteckt, werden dabei zuerst die Federzungen 112 elastisch voneinander weg verformt, da die Rastvorsprünge 111 mit der Oberfläche des Pilzkopfs 155 in Anlage geraten und an diesen entlang gleiten. Wenn die Rastvorsprünge 111 den Pilzkopf 155 passiert haben, verformen sich die Federzungen 112 elastisch zurück in ihre Ausgangsposition und die Rastvorsprünge 111 rasten an der Hinterschneidungsfläche 156 ein. Um diese Rastverbindung zu lösen muss die Sicherheitsplombe 101 gegenüber der Aufnahme 153 verkippt werden, d.h. die Längsachse der Sicherheitsplombe 101 muss einen gewissen Winkel zu der Längsachse der Aufnahme 153 einnehmen. Gleichzeitig muss die Sicherheitsplombe 101 von der Aufnahme 153 gezogen werden.

Durch das Verkippen der Sicherheitsplombe 101 gegenüber der Aufnahme 153 werden die Federzungen 112 ein wenig nach außen verformt, was die Hinterschneidung der Rastvorsprünge 111 mit der Hinterschneidungsfläche 156 weitestgehend aufhebt und somit ein Herunterziehen der Sicherheitsplombe 101 von der Aufnahme 153 ermöglicht.

Der Sicherheitsverschluss 150, an den diese Sicherheitsplombe 101 angepasst ist, ist im Wesentlichen gleich dem Sicherheitsverschluss 50 für die Sicherheitsplombe gemäß dem ersten Ausführungsbeispiel. Wie oben aber bereits erwähnt, muss ein Verdrehen der Sicherheitsplombe 101 um ihre Längsachse herum nicht eingeschränkt werden. Daher kann das Durchgangsloch 157 in dem zweiten Verschlussteil 152 auch kreisförmig ausgebildet sein. Der Durchmesser des Durchgangslochs ist vorzugsweise geringfügig größer als die größte Abmessung des Auerschnitts des Plombenkopfs 120 aber kleiner als die größte Abmessung der äußeren Ränder der beiden Sperrlaschen 121. Dadurch ist eine Überlappung zwischen den Sperrlaschen 121 und dem zweiten Verschlussteil 152 sicher gestellt. Bei diesem Ausführungsbeispiel reicht es somit aus, wenn ein Verkippen der Sicherheitsplombe 101 gegenüber der Aufnahme 153 verhindert ist, damit die Sicherheitsplombe 101 nicht von der Aufnahme 153 entfernt werden kann.

Wenn sich der Sicherheitsverschluss 150 in der Schließstellung befindet, kann die Sicherheitsplombe 101 durch das Durchgangsloch 157 in dem zweiten Verschlussteil 152 auf die Aufnahme 153 aufgesteckt werden, welche an dem ersten Verschlussteil 151 vorgesehen ist. Dabei ist darauf zu achten, dass hinreichend Platz vorgesehen ist, dass sich die Federzungen 112 radial nach außen verformen können. Um eine präzise Positionierung der Sicherheitsplombe 101 entlang ihrer Längsachse zu gewährleisten, ist eine Anlagefläche 158 im Inneren des Plombenkopfs 120 oder des Plombenfußes 110 vorgesehen, die vorteilhafter Weise der Form des Pilzkopfes 155 entspricht. Die Sicherheitsplombe 101 kann nun frei um ihre Längsachse gedreht werden, ohne von der Aufnahme 153 gelöst zu werden. Ein Verkippen der Sicherheitsplombe 101 gegenüber der Aufnahme 153 im verschlossenen Zustand des Sicherheitsverschlusses 150 wird dadurch verhindert, dass das Durchgangsloch 157 in dem zweiten Verschlussteil 152 hinreichend klein ist. Eine geringer Abstand der Unterseiten der Sperrelemente 121 von der Oberfläche des zweiten Verschlussteils 152 stellt zudem zumindest sicher, dass die Sicherheitsplombe 101 nicht verkippt werden kann, ohne die Sicherheitsplombe 101 zu entwerten. Dies gilt selbst für den Fall, dass ein größerer Abstand zwischen der Mantelfläche der Sicherheitsplombe 101 und dem inneren Rand des Durchgangslochs 157 in dem zweiten Verschlussteil 152 vorhanden ist.

Wenn nun der Sicherheitsverschluss 150 geöffnet wird, d.h. das zweite Verschlussteil 152 von dem ersten Verschlussteil 151 weg bewegt wird, werden die Sperrlaschen 121 wir bei dem ersten Ausführungsbeispiel aus ihrer Ausgangsposition ausgelenkt und die Scharnierelemente 122 teilweise plastisch verformt. Bei diesem Ausführungsbeispiel werden die Sperrlaschen 121 nicht durch elastische Federelemente daran gehindert, in ihre Ausgangsposition zurück zu kehren. Allerdings sorgt die plastische Verformung der Scharnierelemente 122 dafür, dass die Sperrlaschen 121 nicht ohne Zwang in dieser Position verbleiben. Auch wenn bei diesem Ausführungsbeispiel keine Stützflächen an der Sicherheitsplombe 101 vorgesehen sind, können die Sperrlaschen 121 trotzdem nicht in der Gegenrichtung so weit verformt werden, dass das Durchgangsloch 157 in dem zweiten Verschlussteil 152 erneut über die Sicherheitsplombe 101 hinweg bewegt werden kann. Dies liegt an dem geringen Abstand zwischen der radial inneren Fläche der Sperrlaschen 121 und der Mantelfläche des Plombenkopfs 120 im Bereich der Unterseite der Sperrlaschen 121. Ein Drängen der Sperrlaschen 121 zu dem Plombenfuß 110 hin führt dazu, dass die radial innere Fläche der Sperrlaschen 121 gegen diese Mantelfläche drückt. Die dadurch erreichbare Verformung der Scharnierelemente 122 ist so gering, dass die plastische Verformung in die Gegenrichtung nicht aufgehoben werden kann.

Auch bei einer mutwilligen Manipulation der Sicherheitsplombe 101 bzw. des Sicherheitsverschlusses 150 ist es zwar möglich, den Verschluss nach einem vorausgehenden Öffnen erneut zu schließen, ohne die Sicherheitsplombe 101 zu entfernen, wie dies zum ersten Ausführungsbeispiel bereits beschrieben war, aber es ist nicht möglich, die Sperrelemente 121 in ihre Ausgangsposition zurück zu bringen und so die entwertete Sicherheitsplombe 101 wie eine nicht entwertete Sicherheitsplombe 101 aussehen zu lassen. Die Sperrlaschen 121 werden deutlich gegenüber dem Plombenkopf 120 der Sicherheitsplombe 101 hervorstehen und so ganz eindeutig anzeigen, dass die Sicherheitsplombe 101 entwertet wurde.

Auch der Sicherheitsverschluss 150 und die Sicherheitsplombe 101 gemäß dem zweiten Ausführungsbeispiel können für einen Sicherheitsbehälter wie beispielsweise einen medizinischen Sterilisierbehälter verwendet werden, wie dies in Fig. 13A und B gezeigt ist.

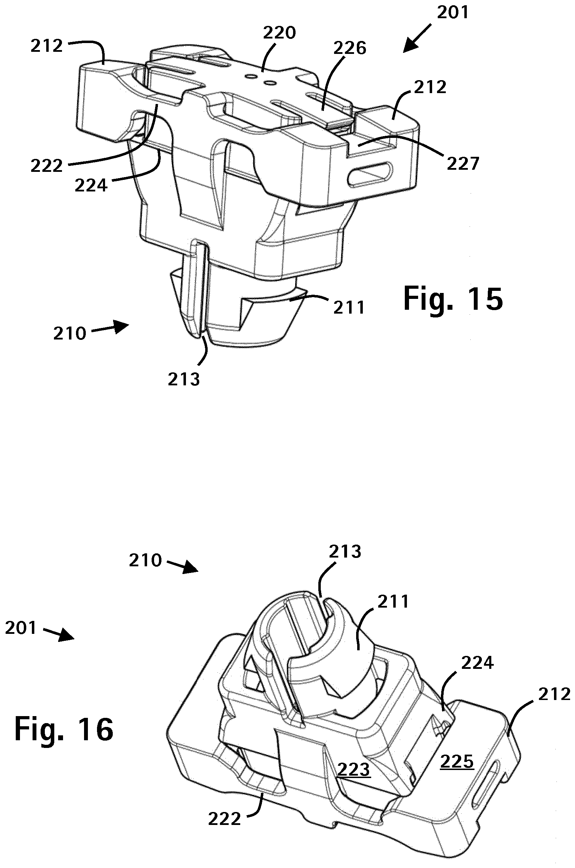

Im Folgenden ist ein drittes Ausführungsbeispiel der vorliegenden Erfindung unter Bezugnahme auf die Fig. 15 und 16 beschrieben.

Die Sicherheitsplombe 201 gemäß dem dritten Ausführungsbeispiel zeigt eine Abwandlung der Sicherheitsplombe 1 gemäß dem ersten Ausführungsbeispiel. Daher sind im Folgenden nur die Unterschiede gegenüber der Sicherheitsplombe des ersten Ausführungsbeispiels erklärt.

Der Plombenfuß 210 ist mit zwei Schlitzen 213 ausgebildet, wodurch das distale Ende des Plombenfußes nach innen verformt werden kann. Auf diese Weise muss nicht die Aufnahme für den Plombenfuß elastisch nachgiebig ausgebildet sein, sondern die Sicherheitsplombe 201 kann in die entsprechende Aufnahme eingerastet werden, indem die beiden Hälften des Plombenfußes elastisch nach innen verformt werden, bis die Rastvorsprünge 211 die Hinterschneidung mit dem entsprechenden Bereich herstellen.

Gemäß diesem Ausführungsbeispiel kann eine größere Hinterschneidung zwischen den Rastvorsprüngen 211 und der Aufnahme hergestellt werden. Bei dem ersten Ausführungsbeispiel ohne Schlitz 213 verformt sich der gesamte Plombenfuß 10 einschließlich der Rastvorsprünge 11.

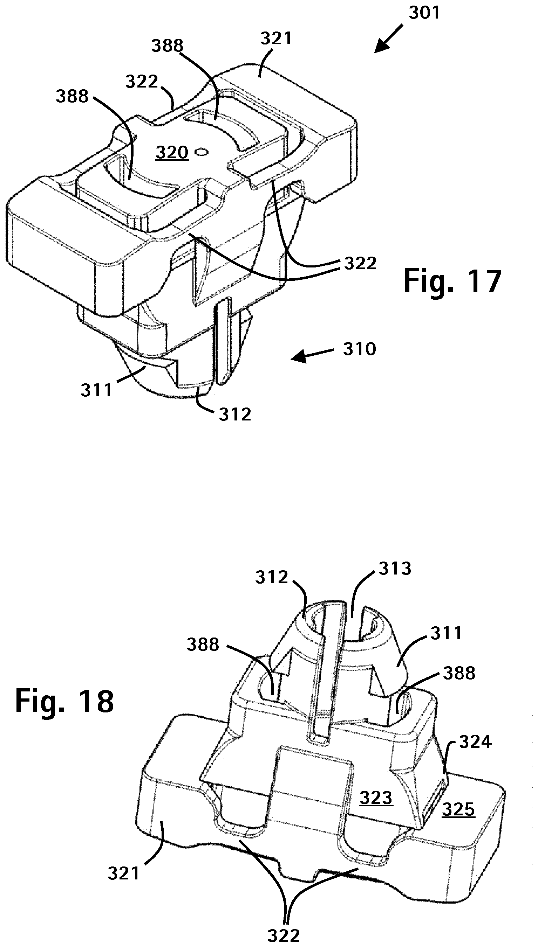

Im Folgenden ist ein viertes Ausführungsbeispiel der vorliegenden Erfindung unter Bezugnahme auf die Fig. 17 und 18 beschrieben.

Die Sicherheitsplombe 201 gemäß dem vierten Ausführungsbeispiel zeigt eine Abwandlung der Sicherheitsplombe 1 gemäß dem ersten und dritten Ausführungsbeispiel. Daher sind im Folgenden nur die Unterschiede gegenüber der Sicherheitsplombe des dritten Ausführungsbeispiels erklärt.

Die Sicherheitsplombe 301 verfügt nicht über elastische Federzungen am Plombenkopf 320, die ein Zurückkehren der Sperrlaschen 312 in die Ausgangsposition zu verhindern, wenn diese aus der Ausgangsposition ausgelenkt wurden. Dies wird bei deiesem Ausführungsbeispiel einzig durch die plastische Verformung, also den Weißbruch, der Scharnierelemente 322 gewährleistet.

Darüber hinaus sind für eine einfachere Ausbildung der Rastvorsprünge 311 Durchgangslöcher 388 in dem Plombenkopf 320 vorgesehen, sodass dort hindurch Teile der Gussformen geführt werden können. Die Rastvorsprünge 311 der Sicherheitsplombe 301 müssen somit nicht zwangsentformt werden. Wenn zudem auf die Stützflächen 324 verzichtet wird, kann grundsätzlich ein Herstellungsverfahren ohne Zwangsentformung eingesetzt werden.

Selbstverständlich können die Merkmale der vorstehend beschriebenen Ausführungsbeispiele auch beliebig geeignet kombiniert werden. Weitere Ausführungsformen ergeben sich dem Fachmann aus den Ansprüchen.

Um einen heimlichen bzw. unauthorisierten Austausch von Sicherheitsplomben zu erkennen, können diese individualisiert sein, beispielsweise durch fortlaufende Nummerierung oder durch eine Unterschrift, Signierung, etc.

Um ein Entwerten der Sicherheitsplomben noch sicherer anzeigen zu können, kann zwischen einer Sperrlasche und dem Plombenkopf eine weitere, vorzugsweise sehr dünne, Verbindung vorgesehen werden, die bei der Bewegung der Sperrlaschen relativ zu dem Plombenkopf zerstört wird. Dazu sollte diese Verbindung entfernt von den Scharnierelementen vorgesehen sein.

## Patentansprüche

1. Sicherheitsplombe (1, 101, 201) mit einem Plombenfuß (10, 110, 210) und einem mit dem Plombenfuß (10, 110, 210) verbundenen Plombenkopf (20, 120, 220) mit wenigstens einer Sperrlasche (21, 121, 221), wobei jede Sperrlasche (21, 121, 221) über wenigstens ein Scharnierelement (22, 122, 222) mit dem Plombenkopf (20, 120, 220) bewegbar verbunden ist,
**dadurch gekennzeichnet, dass**
der Plombenfuß (10,110,210) mindestens zwei Rastvorsprünge (11, 111, 211) und zwei Schlitze (213, 313) aufweist, durch welche das distale Ende des Plombenfußes (10,110,210) nach innen verformbar ist.

2. Sicherheitsplombe (1, 101, 201) nach Anspruch 1, wobei die Sicherheitsplombe (1, 101, 201) einstückig vorzugsweise aus Kunststoff ausgebildet ist und/oder zwei vorzugsweise in radialer Richtung gegenüber liegenden Sperrlaschen (21, 121, 221) aufweist und/oder das wenigstens eine Scharnierelement (22, 122, 222) zumindest teilweise plastisch verformbar ist.

3. Sicherheitsplombe (1, 101, 201) nach einem der vorangehenden Ansprüche, wobei jede Sperrlasche (21, 121, 221) mit zwei Scharnierelementen (22, 122, 222) vorzugsweise seitlich am Plombenkopf (20, 120, 220) vorgesehen ist und/oder zumindest teilweise gegenüber dem Plombenfuß (10, 110, 210) radial vorsteht und/oder der Plombenfuß (10, 110, 210) einen im Wesentlichen kreisförmigen Querschnitt aufweist.

4. Sicherheitsplombe (1, 101, 201) nach einem der vorangehenden Ansprüche, wobei die mindestens zwei Rastvorsprünge (11, 111, 211) in radialer Richtung vom Plombenfuß (10, 110, 210) nach außen vorstehen oder an dem Plombenfuß (10, 110, 210) elastisch gelagert sind und in radialer Richtung nach innen vorstehen.

5. Sicherheitsplombe (1, 101, 201) nach einem der vorangehenden Ansprüche, wobei am Plombenkopf (20, 120, 220) oder am Plombenfuß (10, 110, 210) wenigstens ein radialer Vorsprung oder Absatz (24, 224) ausgebildet ist, an dem die dem Plombenfuß (10, 110, 210) zugewandte Seite mindestens einer Sperrlasche (21, 121, 221) anliegt.

6. Sicherheitsplombe (1, 101, 201) nach einem der vorangehenden Ansprüche, wobei eine Mehrzahl von Rastvorsprüngen (11, 111, 211) vorgesehen ist, die vorzugsweise in gleichen Winkelabständen zueinander um den Plombenfuß (10, 110, 210) herum angeordnet sind, wobei weiter vorzugsweise eine gerade Anzahl von Rastvorsprüngen (11, 111, 211) vorgesehen ist, insbesondere vier oder sechs Rastvorsprünge (11, 111, 211).

7. Sicherheitsplombe (1) nach einem der vorangehenden Ansprüche, wobei an dem Plombenkopf (20) mindestens eine elastische Federzunge (26) vorgesehen ist, die in radialer Richtung über den inneren Rand einer Sperrlasche (21) hinaus hervorsteht, wobei die mindestens eine elastische Federzunge (26) vorzugsweise an der Oberseite des Plombenkopfs (20) und weiter vorzugsweise zentral angebracht ist.

8. Sicherheitsplombe (1, 101, 201) nach einem der vorangehenden Ansprüche, wobei an der Mantelfläche des Plombenkopfs (20, 120, 220) mindestens ein radial vorstehender zweiter Rastvorsprung vorgesehen ist und an mindestens einer Sperrlasche (21, 121, 221) eine Rastnase so angeordnet ist, dass sie dem zweiten Rastvorsprung radial gegenüber liegt und in axialer Richtung im Vergleich zu dem zweiten Rastvorsprung weiter zum Plombenfuß (10, 110, 210) hin angeordnet ist, wobei vorzugsweise wenigstens die Rastnase oder der zweite Rastvorsprung elastisch federnd ausgebildet ist.

9. Sicherheitsverschluss (50, 150) mit
einem ersten Verschlussteil (51, 151), und
einem zweiten Verschlussteil (52, 152), welches zwischen einer Offenstellung und einer Schließstellung relativ zu dem ersten Verschlussteil (51, 151) hin und her bewegbar ist,
wobei das erste Verschlussteil (51, 151) eine Aufnahme (53, 153) aufweist, an der der Plombenfuß (10, 110, 210) einer Sicherheitsplombe (1, 101, 201) nach einem der vorangehenden Ansprüche so einrastbar ist, dass sich, wenn sich das zweite Verschlussteil (52, 152) in der Schließstellung befindet, mindestens eine Sperrlasche (21, 121, 221) der Sicherheitsplombe (1, 101, 201) mit dem zweiten Verschlussteil (52, 152) auf der dem ersten Verschlussteil (51,151) entgegen gesetzten Seite des zweiten Verschlussteils (52, 152) zumindest teilweise überlappt, wobei vorzugsweise das zweite Verschlussteil (52) eine Aussparung und/oder ein Durchgangsloch (53) aufweist und der Plombenfuß (10, 210) dort hindurch führbar und in der Schließstellung des zweiten Verschlussteils (52) in der Plombenfußaufnahme (53) des ersten Verschlussteils (51) einrastbar ist.

10. Sicherheitsverschluss (50, 150) nach Anspruch 9, wobei der Plombenfuß (10, 110, 210) der Sicherheitsplombe (1, 101, 201) in der Schließstellung des zweiten Verschlussteils (52, 152) so an der Plombenfußaufnahme (53, 153) des ersten Verschlussteils (51, 151) einrastbar ist, dass sich die Sicherheitsplombe (1, 101, 201) mit dem zweiten Verschlussteil (52, 152) derart formschlüssig in Anlage befindet, dass die Sicherheitsplombe (1, 101, 201) gegenüber dem ersten Verschlussteil (51, 151) im Wesentlichen unbeweglich gehalten, insbesondere nicht drehbar, kippbar und/oder verschiebbar ist, wobei die Sicherheitsplombe (1, 101, 201) in der Offenstellung des zweiten Verschlussteils (52, 152) gegenüber dem ersten Verschlussteil (51, 151) bewegbar, insbesondere drehbar, kippbar und/oder verschiebbar ist.

11. Sicherheitsverschluss (50) nach Anspruch 9 oder 10, wobei die Aufnahme (53) an dem ersten Verschlussteil (51) mindestens eine Aussparung aufweist, vorzugsweise mit einem im Wesentlichen rotationssymmetrischen Abschnitt mit mindestens zwei radialen Aufweitungen nach außen, in die ein Plombenfuß (10, 210) einer Sicherheitsplombe (1, 201) einführbar ist oder wobei die Aufnahme (53, 153) an dem ersten Verschlussteil (51, 151) eine im Wesentlichen kreisringförmige Aussparung mit mindestens zwei radialen Aufweitungen nach außen und/oder innen ist, in die die mindestens zwei Rastvorsprünge (11, 111, 211) einführbar sind, wobei die Aufnahme (153) an dem ersten Verschlussteil (151) weiter vorzugsweise ein pilzförmiger Vorsprung ist, auf den ein Plombenfuß (110) einer Sicherheitsplombe (101) aufsteckbar ist.

12. Sicherheitsverschluss (50, 150) nach einem der Ansprüche 16 bis 18, der über einen zusätzlichen bekannten Schließmechanismus verfügt, insbesondere über einen Rastmechanismus, mit dem das zweite Verschlussteil (52, 152) an dem ersten Verschlussteil (51, 151) arretierbar ist.

13. Sicherheitsbehälter (70, 170) mit einer Behälterwanne (71, 171), einem Deckel (72, 172) und einem Sicherheitsverschluss (50, 150) nach einem der Ansprüche 9 bis 12, wobei eines von dem ersten und dem zweiten Verschlussteil (51, 151; 52, 152) jeweils an einem von der Behälterwanne (71, 171) und dem Deckel (72, 172) vorgesehen ist wobei vorzugsweise zumindest das erste Verschlussteil (51, 151) oder das zweite Verschlussteil (52, 152) an einer Verschlusslasche vorgesehen ist, welche entsprechend an der Behälterwanne (71, 171) und/oder dem Deckel (72, 172) schwenkbar vorgesehen ist.

14. Sterilisiercontainer (70) mit
einer Containerwanne (71) mit mindestens einem ersten Verschlussteil (51), und
einem Containerdeckel (72), der über mindestens eine Verschlusslasche verfügt, welche schwenkbar an Containerdeckel (72) angebracht ist, sodass sie von einer Offenstellung in eine Schließstellung hin und her bewegbar ist, und einen zweiten Verschlussteil (52) aufweist,
**dadurch gekennzeichnet, dass**
der erste Verschlussteil (51) eine Aussparung (53) aufweist mit zwei gegenüberliegenden kreisbogenförmigen Anlageflächen (55), die an zwei Kreisbogenabschnitten ausgebildet sind, zwischen denen zwei radiale Aufweitungen (57) vorgesehen sind, sodass die Aussparung (53) daran angepasst ist, einen Plombenfuß (10, 201) einer Sicherheitsplombe (1, 201) gemäß einem der Ansprüche 1 bis 8, welcher einen im Wesentlichen kreisförmigen Querschnitt und zwei radial nach außen vorstehende Rastvorsprünge (11, 211) aufweist und in axialer Richtung in die Aussparung (53) einschiebbar ist, so aufzunehmen, dass die Rastvorsprünge (11, 211) eine rastende Hinterschneidung mit den Kreisbogenabschnitten (55) herstellen und eine Mantelfläche des Plombenfußes (10, 210) teilweise in Anlage an die Anlageflächen gelangt, und zudem daran angepasst ist, dass die Rastverbindung zwischen den Rastvorsprüngen (11, 211) und den Kreisbogenabschnitten (55) durch eine Drehung der Sicherheitsplombe (1, 201) um ihre Längsachse um im Wesentlichen 90° aufhebbar ist, sodass die Sicherheitsplombe (1,201) aus der Aussparung (53) entnehmbar ist, und
der zweite Verschlussteil (52)ein Durchgangsloch (54) aufweist, das einen im Wesentlichen nicht rotationssymmetrischen Querschnitt aufweist und daran angepasst ist, einen Plombenkopf (20, 220) der Sicherheitsplombe (1, 201) in Drehrichtung formschlüssig so aufzunehmen, dass eine Bewegung der Verschlusslasche relativ zu der Sicherheitsplombe (1, 201) in Richtung zu der Offenstellung hin zu einer Anlage zumindest eines Teils der Umrandung des Durchgangslochs (54) mit mindestens einer an dem Plombenkopf (20, 220) der Sicherheitsplombe (1, 201) vorgesehenen Sperrlasche (21,221) führt und eine weitergehende Bewegung der Verschlusslasche in dieser Richtung zu einer zumindest teilweisen plastischen Verformung der mindestens einen Sperrlasche (21, 221) führt, sodass das Durchgangsloch (54) der Verschlusslasche über den Plombenkopf (20, 220) hinweg führbar ist.

15. Sterilisiercontainer (70) nach Anspruch 14, wobei zwischen der Verschlusslasche und dem ersten Verschlussteil (51) ein zusätzlicher Verriegelungs- bzw. Verschlussmechanismus vorgesehen ist, sodass der Sicherheitsverschluss (50) in der Schließstellung der Verschlusslasche im Wesentlichen kraftfrei ist.

## Claims

1. Security seal (1, 101, 201) comprising a seal base (10, 110, 210) and a seal head (20, 120, 220), which is connected to the seal base (10, 110, 210) and has at least one locking tab (21, 121, 221), each locking tab (21, 121, 221) being movably connected to the seal head (20, 120, 220) via at least one hinge element (22, 122, 222),
**characterized in that**
the seal base (10, 110, 210) has at least two latching projections (11, 111, 211) and two slots (213, 313) through which the distal end of the seal base (10, 110, 210) can be inwardly deformed.

2. Security seal (1, 101, 201) according to claim 1, wherein the security seal (1, 101, 201) is formed in one piece preferably from plastics material and/or has two preferably radially opposite locking tabs (21, 121, 221), and/or the at least one hinge element (22, 122, 222) is at least partially plastically deformable.

3. Security seal (1, 101, 201) according to any of the preceding claims, wherein each locking tab (21, 121, 221) is provided with two hinge elements (22, 122, 222), preferably at the side on the seal head (20, 120, 220), and/or projects at least partially radially with respect to the seal base (10, 110, 210), and/or the seal base (10, 110, 210) has a substantially circular cross section.

4. Security seal (1, 101, 201) according to any of the preceding claims, wherein the at least two latching projections (11, 111, 211) project radially outward from the seal base (10, 110, 210) or are resiliently mounted on the seal base (10, 110, 210) and project radially inward.

5. Security seal (1, 101, 201) according to any of the preceding claims, wherein at least one radial projection or shoulder (24, 224) is formed on the seal head (20, 120, 220) or on the seal base (10, 110, 210), against which radial projection or shoulder the side of at least one locking tab (21, 121, 221) facing the seal base (10, 110, 210) abuts.

6. Security seal (1, 101, 201) according to any of the preceding claims, wherein a plurality of latching projections (11, 111, 211) is provided which are preferably arranged at equal angular intervals relative to one another around the seal base (10, 110, 210), wherein more preferably an even number of latching projections (11, 111, 211) is provided, in particular four or six latching projections (11, 111, 211).

7. Security seal (1) according to any of the preceding claims, wherein at least one resilient spring tongue (26) is provided on the seal head (20), which spring tongue protrudes radially beyond the inner edge of a locking tab (21), wherein the at least one resilient spring tongue (26) is preferably attached to the upper side of the seal head (20) and more preferably is centrally attached.

8. Security seal (1, 101, 201) according to any of the preceding claims, wherein at least one radially projecting second latching projection is provided on the lateral surface of the seal head (20, 120, 220), and a latching lug is arranged on at least one locking tab (21, 121, 221) such that it is radially opposite the second latching projection and is arranged further toward the seal base (10, 110, 210) in the axial direction by comparison with the second latching projection, wherein preferably at least the latching lug or the second latching projection is designed to be resiliently sprung.

9. Security closure (50, 150) comprising
a first closure part (51, 151), and
a second closure part (52, 152) which can be moved back and forth between an open position and a closed position relative to the first closure part (51, 151),
wherein the first closure part (51, 151) has a receptacle (53, 153) in which the seal base (10, 110, 210) of a security seal (1, 101, 201) according to any of the preceding claims can be latched in such a way that, when the second closure part (52, 152) is in the closed position, at least one locking tab (21, 121, 221) of the security seal (1, 101, 201) is at least partially overlapped by the second closure part (52, 152) on the side of the second closure part (52, 152) opposite the first closure part (51, 151), wherein preferably the second closure part (52) has a recess and/or a through-hole (53), and the seal base (10, 210) can be guided therethrough and can be latched into the seal base receptacle (53) of the first closure part (51) in the closed position of the second closure part (52).

10. Security closure (50, 150) according to claim 9, wherein the seal base (10, 110, 210) of the security seal (1, 101, 201) in the closed position of the second closure part (52, 152) can be latched into the seal base receptacle (53, 153) of the first closure part (51, 151) so that the security seal (1, 101, 201) interlockingly contacts the second closure part (52, 152) such that the security seal (1, 101, 201) is held in a substantially immovable manner, in particular so as not to be rotatable, tiltable and/or displaceable, relative to the first closure part (51, 151), wherein the security seal (1, 101, 201) is movable, in particular is rotatable, tiltable and/or displaceable, relative to the first closure part (51, 151) in the open position of the second closure part (52, 152).

11. Security closure (50) according to claim 9 or 10, wherein the receptacle (53) of the first closure part (51) comprises at least one recess, preferably having a substantially rotationally symmetrical portion having at least two radial widened regions, into which a seal base (10, 210) of a security seal (1, 201) can be inserted, or wherein the receptacle (53, 153) of the first closure part (51, 151) is a substantially toroidal recess having at least two radial widened regions outward and/or inward, into which the at least two latching projections (11, 111, 211) can be inserted, wherein the receptacle (153) of the first closure part (151) is more preferably a mushroom-shaped projection onto which a seal base (110) of a security seal (101) can be placed.

12. Security closure (50, 150) according to any of claims 16 to 18 which has an additional known locking mechanism, in particular a latching mechanism, by means of which the second closure part (52, 152) can be locked to the first closure part (51, 151).

13. Security container (70, 170) comprising a container trough (71, 171), a lid (72, 172) and a security closure (50, 150) according to any of claims 9 to 12, wherein one each of the first and the second closure part (51, 151; 52, 152) is each provided on one of the container trough (71, 171) and the lid (72, 172), wherein preferably at least the first closure part (51, 151) or the second closure part (52, 152) is provided on a closure tab which is provided so as to be correspondingly pivotable on the container trough (71, 171) and/or the lid (72, 172).

14. Sterilizing container (70) comprising
a container trough (71) having at least one first closure part (51), and
a container lid (72) which has at least one closure tab, which is pivotably attached to the container lid (72) so that it can be moved back and forth from an open position into a closed position, and has a second closure part (52),
**characterized in that**
the first closure part (51) has a recess (53) having two opposite circular-arc-shaped contact surfaces (55) which are formed on two circular arc portions, between which two radial widened regions (57) are provided, so that the recess (53) is adapted to receive a seal base (10, 201) of a security seal (1, 201) according to any of claims 1 to 8, which has a substantially circular cross section and two radially outwardly projecting latching projections (11, 211) and can be inserted into the recess (53) in the axial direction, such that the latching projections (11, 211) produce a latching undercut with the circular-arc portions (55) and a lateral surface of the seal base (10, 210) at least partially comes into contact with the contact surfaces, and is also adapted to the fact that the latching connection between the latching projections (11, 211) and the circular arc portions (55) can be released by rotating the security seal (1, 201) about its longitudinal axis by substantially 90° so that the security seal (1, 201) can be removed from the recess (53), and
the second closure part (52) has a through-hole (54) which has a substantially non-rotationally symmetrical cross section and is adapted to form-fittingly receive a seal head (20, 220) of the security seal (1, 201) in the direction of rotation in such a way that a movement of the closure tab relative to the security seal (1, 201) in the direction of the open position leads to contact of at least part of the border of the through-hole (54) with at least one locking tab (21, 221) provided on the seal head (20, 220) of the security seal (1, 201), and a continuing movement of the closure tab in this direction leads to an at least partial plastic deformation of the at least one locking tab (21, 221), so that the through-hole (54) of the closure tab can be guided over the seal head (20, 220).

15. Sterilizing container (70) according to claim 14, wherein an additional locking or closure mechanism is provided between the closure tab and the first closure part (51), so that the security closure (50) is substantially free of force in the closed position of the closure tab.

## Revendications

1. Plomb de sécurité (1, 101, 201) comportant un pied de plomb (10, 110, 210) et une tête de plomb (20, 120, 220) reliée au pied de plomb (10, 110, 210) comportant au moins une languette de verrouillage (21, 121, 221), dans lequel chaque languette de verrouillage (21, 121, 221) est reliée de manière mobile à la tête de plomb (20, 120, 220) par l'intermédiaire d'au moins élément de charnière (22, 122, 222),
**caractérisé en ce que**
le pied de plomb (10, 110, 210) présente au moins deux saillies d'encliquetage (11, 111, 211) et deux fentes (213, 313) à travers lesquelles l'extrémité distale du pied de plomb (10, 110, 210) est déformable vers l'intérieur.

2. Plomb de sécurité (1, 101, 201) selon la revendication 1, dans lequel le plomb de sécurité (1, 101, 201) est réalisé d'un seul tenant préférablement en matière plastique et/ou présente deux languettes de verrouillage (21, 121, 221) opposées préférablement dans la direction radiale et/ou l'au moins un élément de charnière (22, 122, 222) est déformable plastiquement au moins partiellement.

3. Plomb de sécurité (1, 101, 201) selon l'une des revendications précédentes, dans lequel chaque languette de verrouillage (21, 121, 221) comportant deux éléments de charnière (22, 122, 222) est prévue préférablement latéralement sur la tête de plomb (20, 120, 220) et/ou dépasse radialement au moins partiellement par rapport au pied de plomb (10, 110, 210) et/ou le pied de plomb (10, 110, 210) présente une section transversale sensiblement circulaire.

4. Plomb de sécurité (1, 101, 201) selon l'une des revendications précédentes, dans lequel les au moins deux saillies d'encliquetage (11, 111, 211) dépassent vers l'extérieur dans la direction radiale à partir du pied de plomb (10, 110, 210) ou sont montées élastiquement sur le pied de plomb (10, 110, 210) et dépassent vers l'intérieur dans la direction radiale.

5. Plomb de sécurité (1, 101, 201) selon l'une des revendications précédentes, dans lequel au moins une saillie ou un épaulement (24, 224) radial(e) est réalisé(e) sur la tête de plomb (20, 120, 220) ou sur le pied de plomb (10, 110, 210), saillie ou épaulement sur laquelle/lequel le côté faisant face au pied de plomb (10, 110, 210) d'au moins une languette de verrouillage (21, 121, 221) repose.

6. Plomb de sécurité (1, 101, 201) selon l'une des revendications précédentes, dans lequel une pluralité de saillies d'encliquetage (11, 111, 211) est prévue, lesquelles saillies d'encliquetage sont agencées l'une par rapport à l'autre préférablement à intervalles angulaires égaux autour du pied de plomb (10, 110, 210), dans lequel il est en outre prévu préférablement un nombre pair de saillies d'encliquetage (11, 111, 211), en particulier quatre ou six saillies d'encliquetage (11, 111, 211).

7. Plomb de sécurité (1) selon l'une des revendications précédentes, dans lequel, sur la tête de plomb (20), il est prévu au moins une languette élastique (26) qui dépasse en direction radiale le bord intérieur d'une languette de verrouillage (21), dans lequel l'au moins une languette élastique (26) est montée préférablement sur le côté supérieur de la tête de plomb (20) et est montée mieux encore centralement.

8. Plomb de sécurité (1, 101, 201) selon l'une des revendications précédentes, dans lequel il est prévu, sur la surface d'enveloppe de la tête de plomb (20, 120, 220), au moins une seconde saillie d'encliquetage dépassant radialement et, sur au moins une languette de verrouillage (21, 121, 221), est agencé un ergot d'encliquetage, de sorte que celui-ci se trouve radialement opposé par rapport à la seconde saillie d'encliquetage et est agencé plus loin vers le pied de plomb (10, 110, 210) dans la direction axiale en comparaison à la seconde saillie d'encliquetage, dans lequel au moins l'ergot d'encliquetage ou la seconde saillie d'encliquetage est préférablement réalisé(e) de manière élastique.

9. Serrure de sécurité (50, 150), comportant
un premier organe de fermeture (51, 151), et
un second organe de fermeture (52, 152) mobile en va-et-vient entre une position ouverte et une position fermée par rapport au premier organe de fermeture (51, 151),
dans laquelle le premier organe de fermeture (51, 151) présente un logement (53, 153) dans lequel le pied de plomb (10, 110, 210) d'un plomb de sécurité (1, 101, 201) selon l'une des revendications précédentes peut être encliqueté de sorte que, lorsque le second organe de fermeture (52, 152) se trouve dans la position fermée, au moins une languette de verrouillage (21, 121, 221) du plomb de sécurité (1, 101, 201) chevauche au moins partiellement le second organe de fermeture (52, 152) sur le côté du second organe de fermeture (52, 152) qui est opposé au premier organe de fermeture (51, 151), dans laquelle préférablement le second organe de fermeture (52) présente un évidement et/ou un trou traversant (53) et le pied de plomb (10, 210) peut être guidé à travers celui-ci et être encliqueté dans la position fermée du second organe de fermeture (52) dans le logement de pied de plomb (53) du premier organe de fermeture (51).

10. Serrure de sécurité (50, 150) selon la revendication 9, dans laquelle le pied de plomb (10, 110, 210) du plomb de sécurité (1, 101, 201) dans la position fermée du second organe de fermeture (52, 152) peut être encliqueté dans le logement de pied de plomb (53, 153) du premier organe de fermeture (51, 151), de sorte que le plomb de sécurité (1, 101, 201) se trouve en appui par complémentarité de forme sur le second organe de fermeture (52, 152), de telle sorte que le plomb de sécurité (1, 101, 201) est maintenu sensiblement stationnaire, en particulier non rotatif, non inclinable et/ou non déplaçable, par rapport au premier organe de fermeture (51, 151), dans laquelle le plomb de sécurité (1, 101, 201) est mobile, en particulier rotatif, inclinable et/ou déplaçable, par rapport au premier organe de fermeture (51, 151), dans la position ouverte du second organe de fermeture (52, 152).

11. Serrure de sécurité (50) selon la revendication 9 ou 10, dans laquelle le logement (53) sur le premier organe de fermeture (51) présente au moins un évidement, comportant préférablement une section sensiblement symétrique en rotation, laquelle section comporte au moins deux évasements radiaux vers l'extérieur, dans lesquels un pied de plomb (10, 210) d'un plomb de sécurité (1, 201) peut être inséré ou dans laquelle le logement (53, 153) sur le premier organe de fermeture (51, 151) est un évidement sensiblement annulaire comportant au moins deux évasements radiaux vers l'extérieur et/ou vers l'intérieur, évasements dans lesquels les au moins deux saillies d'encliquetage (11, 111, 211) peuvent être insérées, dans laquelle le logement (153) sur le premier organe de fermeture (151) est au mieux une saillie en forme de champignon, sur laquelle un pied de plomb (110) d'un plomb de sécurité (101) peut être emboîté.

12. Serrure de sécurité (50, 150) selon l'une des revendications 16 à 18, laquelle dispose d'un mécanisme de fermeture connu supplémentaire, en particulier d'un mécanisme à encliquetage à l'aide duquel le second organe de fermeture (52, 152) est verrouillable sur le premier organe de fermeture (51, 151).

13. Enceinte de confinement (70, 170) comportant une cuve d'enceinte (71, 171), un couvercle (72, 172) et une serrure de sécurité (50, 150) selon l'une des revendications 9 à 12, dans laquelle l'un parmi le premier et le second organe de fermeture (51, 151 ; 52, 152) est prévu respectivement sur l'un des éléments parmi la cuve d'enceinte (71, 171) et le couvercle (72, 172), dans lequel préférablement au moins le premier organe de fermeture (51, 151) ou le second organe de fermeture (52, 152) est prévu sur une languette de fermeture qui est de manière correspondante prévue oscillante sur la cuve d'enceinte (71, 171) et/ou sur le couvercle (72, 172).

14. Container de stérilisation (70), comportant
une cuve de container (71) comportant au moins un premier organe de fermeture (51), et
un couvercle de container (72) qui dispose d'au moins une languette de fermeture montée oscillante sur le couvercle de container (72) de manière à ce qu'elle soit mobile en va-et-vient d'une position ouverte à une position fermée, et présente un second organe de fermeture (52),
**caractérisé en ce que**
le premier organe de fermeture (51) présente un évidement (53) comportant deux surfaces d'appui (55) opposées ayant une forme d'arc circulaire, qui sont réalisées sur deux sections d'arcs circulaires entre lesquelles deux évasements (57) radiaux sont prévus, de manière à ce que l'évidement (53) soit adapté pour recevoir un pied de plomb (10, 201) d'un plomb de sécurité (1, 201) selon l'une des revendications 1 à 8, lequel pied de plomb présente une section transversale sensiblement circulaire et deux saillies d'encliquetage (11, 211) dépassant radialement vers l'extérieur et peut être inséré axialement dans l'évidement (53), de sorte que les saillies d'encliquetage (11, 211) forment une contre-dépouille encliquetable avec les sections d'arcs circulaires (55) et qu'une surface d'enveloppe du pied de plomb (10, 210) vienne partiellement en appui sur les surfaces d'appui, et soit en outre adapté de façon à ce que la liaison à encliquetage entre les saillies d'encliquetage (11, 211) et les sections d'arcs circulaires (55) puisse être supprimée par une rotation du plomb de sécurité (1, 201) de 90° sensiblement autour de l'axe longitudinal de celui-ci, de manière à ce que le plomb de sécurité (1, 201) puisse être retiré de l'évidement (53), et
le second organe de fermeture (52) présente un trou traversant (54), qui présente une section transversale sensiblement non symétrique en rotation et est adapté pour recevoir par complémentarité de forme une tête de plomb (20, 220) du plomb de sécurité (1, 201) dans le sens de rotation, de sorte qu'un mouvement de la languette de fermeture par rapport au plomb de sécurité (1, 201) en direction de la position ouverte entraîne un appui d'au moins une partie de la bordure du trou traversant (54) avec au moins une languette de verrouillage (21, 221) prévue sur la tête de plomb (20, 220) du plomb de sécurité (1, 201) et qu'un mouvement supplémentaire de la languette de fermeture dans cette direction entraîne une déformation plastique au moins partielle de l'au moins une languette de verrouillage (21, 221), de manière à ce que le trou traversant (54) de la languette de fermeture puisse être guidé au-delà de la tête de plomb (20, 220).

15. Container de stérilisation (70) selon la revendication 14, dans lequel un mécanisme de verrouillage ou de fermeture supplémentaire est prévu entre la languette de fermeture et le premier organe de fermeture (51), de manière à ce que la serrure de sécurité (50) soit sensiblement exempte de force dans la position fermée de la languette de fermeture.
